(19) Europäisches Patentamt
European Patent Office
Office européen des brevets

(11) Publication number: 0 328 669 A1

(12) **EUROPEAN PATENT APPLICATION published in accordance with Art. 158(3) EPC**

(21) Application number: 88906128.9

(51) Int. Cl.[4]: C07C 149/32

(22) Date of filing: 23.07.88

(86) International application number: PCT/JP88/00744

(87) International publication number: WO 89/00989 (09.02.89 89/04)

(30) Priority: 25.07.87 JP 184684/87
14.10.87 JP 257227/87

(43) Date of publication of application: 23.08.89 Bulletin 89/34

(84) Designated Contracting States: DE FR GB

(71) Applicant: NIPPON OIL AND FATS COMPANY, LIMITED
10-1, Yuraku-cho 1-chome
Chiyoda-ku Tokyo100(JP)

(72) Inventor: HAYASHI, Akio
2-6-3, Amakubo Tsukuba-shi
Ibaraki 305(JP)
Inventor: GOTO, Yoshitaka
2-24-5, Umezono Tsukuba-shi
Ibaraki 305(JP)
Inventor: NAKAYAMA, Masaharu
2-15-5, Umezono Tsukuba-shi
Ibaraki 305(JP)

(74) Representative: Kraus, Walter, Dr. et al
Patentanwälte Kraus, Weisert & Partner
Thomas-Wimmer-Ring 15
D-8000 München 22(DE)

# (54) CHALCONE DERIVATIVE COMPOUNDS.

(57) Chalcone derivative compounds represented by general formula (I) or (II), wherein $R_1$ and $R_2$ each represents a halogen atom, a hydroxy group, an amino group, a dimethylamino group, a nitro group, a cyano group, a phenyl group, an acetyl group, an alkyl group containing 1 to 18 carbon atoms or an alkyloxy group containing 1 to 22 carbon atoms, $n_1$ and $n_2$ each represents an integer of 0 to 21, and $m_1$ and $m_2$ each represents an integer of 0 to 5.

EP 0 328 669 A1

# Chalcone Derivative Compound

## Field of Art

This invention relates to a chalcone derivative compound that may be utilized in, for example, pharmaceuticals, agricultural chemicals, antibacterial substances, insecticides or repellents.

## Background of Art

Up to the present time, extensive studies have been made on the physiological activities of compounds having a chalcone skeleton or a skeleton similar to that of chalcone, whether the compounds are synthesized products or of natural origin. The antibacterial properties exhibited by, for example, penicillic acid, are thought to be ascribable to $\alpha$, $\beta$-unsaturated ketone as a component of the molecular structure of penicillic acid, and similar effects are expected from the chalcone derivative compounds having the comparable structure. Also, extensive researches have been conducted into physiological activities of the compounds having the chalcone skeleton. Of these, as the chalcones having hetero atoms, there are known for example azachalcones, that is, the nitrogen-containing chalcone derivative compounds. These azachalcones have a structure in which the phenyl group of chalcone is substituted by a pyridyl group, and a number of reports have been made on their pharmacological properties, including anti-cancerous properties.

As for the chalcone derivative compounds having hetero atoms other than nitrogen atoms, for example, sulfur-containing chalcone derivative compounds, the number of reports on these compounds is few. According to a "YAKUGAKU ZASHI" by Hirose et al., vol.91, No.8, p.804 (1971), tests on synthesis and anti-bacterial properties have been conducted on a series of compounds having an aromatic ring with a methylthio group in a benzoyl moiety of a chalcone skeleton. As a result, it has been reportedly revealed that superior anti-bacterial properties are displayed when the methylthio group present in the aromatic ring of the benzoyl moiety is at the ortho position.

However, although the substituents in the benzylidene moieties of a series of 4′-methylthiochalcone derivatives and 2′-methylthiochalcone derivatives tested for anti-bacterial properties are exclusively at the ortho positions, it cannot be necessarily concluded that the ortho substituents give rise to most favorable results.

It is an object of the present invention to provide a chalcone derivative compound that may be utilized in, for example, pharmaceuticals, agricultural chemicals, anti-bacterial substances, insecticides or repellents.

## Disclosure of the Invention

According to the present invention, there is provided a chalcone derivative compound represented by the following general formula (I)

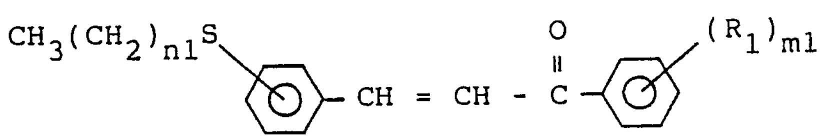 ... (I)

wherein $R_1$ represents a halogen atom, a hydroxyl group, an amino group, a dimethylamino group, a nitro group, a cyano group, a phenyl group, an acetyl group, an alkyl group having 1 to 18 carbon atoms or an alkyloxy group having 1 to 22 carbon atoms, $n_1$ represents an integer of 0 to 21 and $m_1$ represents an integer of 0 to 5.

According to the present invention, there is also provided a chalcone derivative compound represented by the following general formula (II)

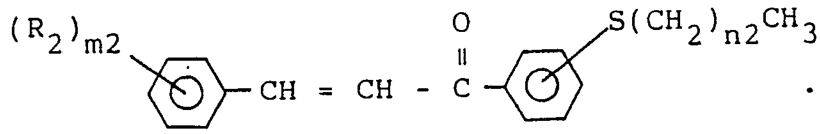

... (II)

wherein $R_2$ represents a halogen atom, a hydroxyl group an amino group, a dimethylamino group, a nitro group, a cyano group, a phenyl group, an acetyl group, an alkyl group having 1 to 18 carbon atoms or an alkyloxy group having 1 to 22 carbon atoms, $n_2$ represents an integer of 0 to 21 and $m_2$ represents an integer of 0 to 5.

Brief Description of the Drawings

Fig.1 is a chart showing the infrared (IR) spectrum of a purified product of 4-methylthiochalcone among the chalcone derivative compounds of the present invention;

Fig.2 is a chart showing the nuclear magnetic resonance (NMR) spectrum of the purified product of 4-methylthiochalcone;

Fig.3 is a chart showing the NMR spectrum of a purified product of 4-butylthio-3′-iodochalcone; and

Fig.4 is a chart showing the NMR spectrum of a purified product of 3-bromo-4′-methylthiochalcone.

Preferred Embodiments to Practice the Invention

The present invention will be explained in detail hereinbelow.

In the following general formula (I)

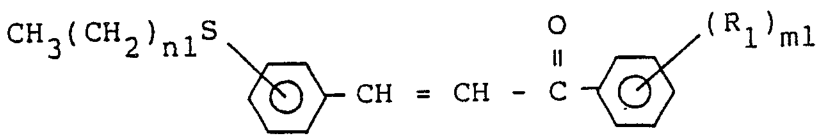

... (I)

of the present invention, $R_1$ represents a halogen atom, a hydroxyl group, an amino group, a dimethylamino group, a nitro group, a cyano group, a phenyl group, an acetyl group, an alkyl group having 1 to 18 carbon atoms or an alkyloxy group having 1 to 22 carbon atoms, $n_1$ represents an integer of 0 to 21 and $m_1$ represents an integer of 0 to 5.

Also, in the following general formula (II)

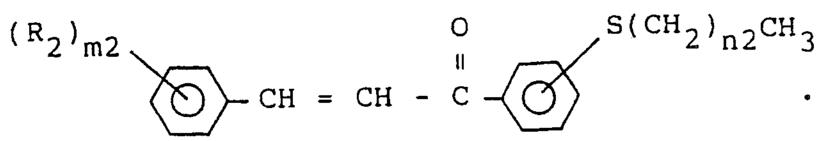

... (II)

of the present invention, $R_2$ represents a halogen atom, a hydroxyl group, an amino group, a dimethylamino group, a nitro group, a cyano group, a phenyl group, an acetyl group, an alkyl group having 1 to 18 carbon atoms or an alkyloxy group having 1 to 22 carbon atoms, $n_2$ represents an integer of 0 to 21 and $m_2$ represents an integer of 0 to 5. When $n_1$ or $n_2$ is not less than 22, difficulties are presented in manufacture, so that the invention cannot be applied. When $R_1$ and $R_2$ each represent an alkyl group having not less than 19 carbon atoms or an alkyloxy group having not less than 23 carbon atoms, difficulties are presented in manufacture of the feed material, so that tha invention cannot similarly be applied. Thus, according to the present invention, there is provided a novel chalcone derivative having an aromatic ring with an alkylthio group in the benzylidene moiety and having a variable substituent in the aromatic ring of the benzoyl moiety of the chalcone skeleton, or a novel chalcone derivative having an aromatic ring with an alkylthio group in the benzoyl moiety and having a variable substituent in the aromatic ring of the benzylidene moiety of the chalcone skeleton.

According to the present invention, the chalcone derivative compound having the above general formula (I) may be produced by the condensation reaction between alkylthiobenzaldehyde and acetophenone or derivatives thereof in the presence of a basic or acidic catalyst. Similarly, the chalcone derivative compound having the above formula (II) may be produced by the condensation reaction between alkylthioacetophenone derivatives and benzaldehyde or derivatives thereof in the presence of a basic or acidic catalyst. As the basic catalysts, sodium hydroxide, potassium hydroxide or various quaternary ammonium salts, for example, may be employed and, as the acidic catalysts, boron trifluoride, phosphorus oxychloride or boron trifluoride etherate, for example, may be employed. The condensation reaction is preferably carried out in the temperature range of from 0° to 50° C for 30 minutes to 25 hours using suitable solvents, such as, for example, organic solvents exhibiting higher polarities, more specifically, alcohols such as methanol, ethanol or propanol. In carrying out the reaction, the reaction temperature higher than 50° C is not preferred because various secondary reactions are caused by heat. On the other hand, the reaction temperature lower than 0° C is also not preferred because the reaction time is excessively prolonged.

Acetophenone and desirable acetophenone derivatives, employed in the chalcone derivative compound represented by the general formula (I) of the present invention, may include, for example, acetophenone, 3-chloroacetophenone, 4-chloroacetophenone, 2,4-dichloroacetophenone, 2,4,6-trichloroacetophenone, 2,3,4,5-tetrachloroacetophenone, 2,3,4,5,6-pentachloro acetophenone, 4-fluoroacetophenone, 2,3-difluoroacetophenone, 2,5-difluoroacetophenone, 2,6-difluoroacetophenone, 3,4,5-trifluoroacetophenone, 2,4,6-trifluoroacetophenone, 2,3,4,6-tetrafluoroacetophenone, 2,3,5,6-tetrafluoroacetophenone, 2,3,4,5,6-pentafluoroacetophenone, 2-bromoacetophenone, 4-bromoacetophenone, 2,5-dibromoacetophenone, 2,3,4-tribromoacetophenone, 2,4,6-tribromoacetophenone, 2-iodoacetophenone, 4-iodoacetophenone, 3,5-diiodoacetophenone, 2,3,4-triiodoacetophenone, 2,4,6-triiodoacetophenone, 2-hydroxyacetophenone, 3-hydroxyacetophenone, 4-hydroxyacetophenone, 2,3-dihydroxyacetophenone, 2,4-dihydroxyacetophenone, 2,6-dihydroxyacetophenone, 3,4-dihydroxyacetophenone, 2,4,5-trihydroxyacetophenone, 3,4,5-trihydroxyacetophenone, 2,3,4,5-tetrahydroxyacetophenone, 2,3,4,5,6-pentahydroxyacetophenone, 4-methylacetophenone, 2,3-dimethylacetophenone, 2,6-dimethylacetophenone, 3,5-dimethylacetophenone, 2,3,4-trimethylacetophenone, 2,4,5-trimethylacetophenone, 2,4,6-trimethylacetophenone, 4-ethylacetophenone, 2,3-diethylacetophenone, 3,4-diethylacetophenone, 3,5-diethylacetophenone, 2,3,5-triethylacetophenone, 4-propylacetophenone, 2,3-dipropylacetophenone, 3,4,5-tripropylacetophenone,4-buthylacetophenone, 2-pentylacetophenone, 3-pentylacetophenone, 4-pentylacetophenone, 4-hexylacetophenone, 2-aminoacetophenone, 3-aminoacetophenone, 4-aminoacetophenone, 4-dimethylaminoacetophenone, 2-nitroacetophenone, 3-nitroacetophenone, 4-nitroacetophenone, 3,5-dinitroacetophenone, 3-phenylacetophenone, 4-phenylacetophenone, 3-acetylacetophenone, 4-acetylacetophenone, 3,5-diacetylacetophenone, 2-methoxyacetophenone, 3-methoxyacetophenone, 4-methoxyacetophenone, 2,3-dimethoxyacetophenone, 2,4-dimethoxyacetophenone, 2,5-dimethoxyacetophenone, 2,6-dimethoxyacetophenone, 3,4-dimethoxyacetophenone, 3,5-dimethoxyacetophenone, 2,3,4-trimethoxyacetophenone, 2,3,5-trimethoxyacetophenone, 2,3,6-trimethoxyacetophenone, 2,4,5-trimethoxyacetophenone, 2,4,6-trimethoxyacetophenone, 3,4,5-trimethoxyacetophenone, 2,3,4,5-tetramethoxyacetophenone, 2,3,4,6-tetramethoxyacetophenone, 2,3,5,6-tetramethoxyacetophenone, 2,3,4,5,6-pentamethoxyacetophenone, 2-ethoxyacetophenone, 3-ethoxyacetophenone, 4-ethoxyacetophenone, 2,3-diethoxyacetophenone, 2,4-diethoxyacetophenone, 2,5-diethoxyacetophenone, 2,6-diethoxyacetophenone, 2,3,4-triethoxyacetophenone, 2,3,5-triethoxyacetophenone, 2,4,5-triethoxyacetophenone, 4-propyloxyacetophenone, 2-butoxyacetophenone, 3-butoxyacetophenone, 4-butoxyacetophenone, 3,4-dibutoxyacetophenone, 3,5-dibutoxyacetophenone, 3,4,5-tributoxyacetophenone, 4-pentyloxyacetophenone, 3,4,5-tripentyloxyacetophenone, 4-hexyloxyacetophenone, 3,4-dihexyloxyacetophenone, 3,5-dihexyloxyacetophenone, 4-heptyloxyacetophenone, 3,4-diheptyloxyacetophenone, 3,4,5-triheptyloxyacetophenone, 4-octyloxyacetophenone, 3,4,5-trioctyloxyacetophenone, 4-nonyloxyacetophenone, 3,4-dinonyloxyacetophenone, 3,4,5-trinonyloxyacetophenone, 2-decyloxyacetophenone, 4-decyloxyacetophenone, 3,4-didecyloxyacetophenone, 3,4,5-tridecyloxyacetophenone, 3-dodecyloxyacetophenone, 4-dodecyloxyacetophenone, 4-tridecyloxyacetophenone, 4-tetraoxyacetophenone, 3,4-di(pentadecyloxy)acetophenone, 3,5-di(pentadecyloxy)acetophenone, 4-hexadecyloxyacetophenone, 3-heptadecyloxyacetophenone, 4-heptadecyloxyacetophenone, 3,5-di(heptadecyloxy)acetophenone, 3-octadecyloxyacetophenone, 4-octadecyloxyacetophenone, 3,4-di(octadecyloxy)acetophenone, 3,4,5-tri(octadecyloxy)acetophenone, 4-nonadecyloxyacetophenone, 3,4-di(nonadecyloxy)acetophenone, 3-eicosyloxyacetophenone, 4-eicosyloxyacetophenone, 3-heneicosyloxyacetophenone or 4-heneicosyloxyacetophenone.

The chalcone derivative compounds obtained in accordance with the present invention and represented by the aforementioned general formula (I) preferably include, for example 4-methylthiochalcone, 4-methylthio-4′-chlorochalcone, 4-methylthio-4′-bromochalcone, 4-methylthio-4′-hydroxychalcone, 4-methylthio-4'-nitrochalcone, 4-methylthio-4'-aminochalcone, 4-methylthio-4'-dimethylaminochalcone, 4-methylthio-4'-methylchalcone, 4-methylthio-4'-ethylchalcone, 4-methylthio-4'-butylchalcone, 4-methylthio-4'-hexylchalcone, 4-methylthio-4'-octylchalcone, 4-methylthio-4'-decylchalcone, 4-methylthio-4'-dodecylchalcone, 4-methylthio-4'-tetradecylchalcone, 4-methylthio-4'-hexadecylchalcone, 4-methylthio-4'-octadecylchalcone, 4-methylthio-4'-methoxychalcone, 4-methylthio-4'-ethoxychalcone, 4-methylthio-4'-butoxychalcone, 4-methylthio-4'-hexyloxychalcone, 4-methylthio-4'-octyloxychalcone, 4-methylthio-4'-decyloxychalcone, 4-methylthio-4'-dodecyloxychalcone, 4-methylthio-4'-tetradecyloxychalcone, 4-methylthio-4'-hexadecyloxychalcone, 4-methylthio-4'-octadecyloxychalcone, 4-methylthio-3'-chlorochalcone, 4-methylthio-3'-bromochalcone, 4-methylthio-3'-hydroxychalcone, 4-methylthio-3'-nitrochalcone, 4-methylthio-3'-aminochalcone, 4-methylthio-3'-dimethylaminochalcone, 4-methylthio-3'-methylchalcone, 4-methylthio-3'-ethylchalcone, 4-methylthio-3'-butylchalcone, 4-methylthio-3'-hexylchalcone, 4-methylthio-3'-octylchalcone, 4-methylthio-3'-decylchalcone, 4-methylthio-3'-dodecylchalcone, 4-methylthio-3'-tetradecylchalcone, 4-methylthio-3'-hexadecylchalcone, 4-methylthio-3'-octadecylchalcone, 4-methylthio-3'-methoxychalcone, 4-methylthio-3'-ethoxychalcone, 4-methylthio-3'-butoxychalcone, 4-methylthio-3'-hexyloxy chalcone, 4-methylthio-3'-octyloxychalcone, 4-methylthio-3'-decyloxychalcone, 4-methylthio-3'-dodecyloxychalcone, 4-methylthio-3'-tetradecyloxychalcone, 4-methylthio-3'-hexadecyloxychalcone, 4-methylthio-3'-octadcyloxychalcone, 4-methylthio-2'-nitrochalcone, 4-methylthio-3',4'-dimethoxychalcone, 4-methylthio-3',4',5'-trimethoxychalcone, 4-methylthio-4'-phenylchalcone, 4-methylthio-4'-acetylchalcone, 4-butylthiochalcone, 4-butylthio-3'-chlorochalcone, 4-butylthio-4'-chlorochalcone, 4-butylthio-2',4'-dichlorochalcone, 4-butylthio-2',4',6'-trichlorochalcone, 4-pentylthio-2',3',4',5'-tetrachlorochalcone, 4-pentylthio-2',3',4',5',6'-pentachlorochalcone, 4-pentylthio-4'-fluorochalcone, 4-pentylthio-2',3'-difluorochalcone, 4-pentylthio-2',5'-difluorochalcone, 4-hexylthio-2',6'-difluorochalcone, 4-hexylthio-3',4',5'-trifluorochalcone, 4-hexylthio-2',4',6'-trifluorochalcone, 4-hexylthio-2',3',4',6'-tetrafluorochalcone, 4-hexylthio-2',3',5',6'-tetrafluorochalcone, 4-hexylthio-2',3',4',5',6'-pentafluorochalcone, 4-heptylthio-2'-bromochalcone, 4-heptylthio-4'-bromochalcone, 4-heptylthio-2',5'-dibromochalcone, 4-heptylthio-2',3',4'-tribromochalcone, 4-heptylthio-2',4',6'-tribromochalcone, 4-octylthio-2'-iodo chalcone, 4-octylthio-4'-iodochalcone, 4-octylthio-3',5'-diiodochalcone, 4-octylthio-2',3',4'-triiodochalcone, 4-octylthio-2',4',6'-triiodochalcone, 4-decylthio-3'-hydroxychalcone, 4-decylthio-4'-hydroxychalcone, 4-decylthio-2',3' dihydroxychalcone, 4-decylthio-2',4'-dihydroxychalcone, 4-decylthio-2',6'-dihydroxychalcone, 4-decylthio-3',4'-dihydroxychalcone, 4-undecylthio-2',4',5'-trihydroxychalcone, 4-undecylthio-3',4',5'-trihydroxyghchalcone, 4-undecylthio-2',3',4',5'-tetrahydroxychalcone, 4-undecylthio-2',3',4',5',6'-pentahydroxychalcone, 4-undecylthio-2',3'-dimethylchalcone, 4-dodecylthio-2',6'-dimethylchalcone, 4-dodecylthio-3',5'-dimethylchalcone, 4-dodecylthio-2',3',4'-trimethylchalcone, 4-dodecylthio-2',4',5'-trimethylchalcone, 4-dodecylthio-2',4',6'-trimethylchalcone, 4-tridecylthio-4'-ethylchalcone, 4-tridecylthio-2',3'-diethylchalcone, 4-tridecylthio-3',4'-diethylchalcone, 4-tridecylthio-3',5'-diethylchalcone, 4-tridecylthio-2',3',5'-triethylchalcone, 4-tetradecylthio-4'-propylchalcone, 4-tetradecylthio-2',3'-dipropylchalcone, 4-tetradecylthio-3',4',5'-tripropylchalcone, 4-pentadecylthio-4'-butyl chalcone, 4-pentadecylthio-2'-pentylchalcone, 4-pentadecylthio-3'-pentylchalcone, 4-pentadecylthio-4'-hexylchalcone, 4-pentadecylthio-2'-aminochalcone, 4-pentadecylthio-3'-aminochalcone, 4-hexadecylthio 3'-nitrochalcone, 4-hexadecylthio-3',5'-dinitrochalcone, 4-hexadecylthio-3'-phenylchalcone, 4-hexadecylthio-4'-phenylchalcone, 4-heptadecylthio-3'-acetylchalcone, 4-heptadecylthio-3',5'-diacetylchalcone, 4-heptadecylthio-3'-methoxychalcone, 4-heptadecylthio-2',3'-dimethoxychalcone, 4-heptadecylthio-2',4'-methoxychalcone, 4-heptadecylthio-2',5'-dimethoxychalcone, 4-heptadecylthio-2',6'-dimethoxychalcone, 4-heptadecylthio-3',4'-dimethoxychalcone, 4-heptadecylthio-3',5'-dimethoxychalcone, 4-octadecylthio-2',3',4'-trimethoxychalcone, 4-octadecylthio-2'3',5'-trimethoxychalcone, 4-octadecylthio-2',3',6'-trimethoxychalcone, 4-octadecylthio-2',4',5'-trimethoxychalcone, 4-octadecylthio-2',4',6'-trimethoxychalcone, 4-octadecylthio-3',4',5'-trimethoxychalcone, 4-otadecylthio-2',3',4',5'-tetramethoxychalcone, 4-octadecylthio-2',3',4',6'-tetramethoxychalcone, 4-octadecylthio-2',3',5',6'-tetramethoxychalcone, 4-octadecylthio-2',3',4',5',6'-pentamethoxychalcone, 4-nonadecylthio-2'-ethoxychalcone, 4-nonadecyl thio-3'-ethoxychalcone, 4-nonadecylthio-4'-ethoxychalcone, 4-nonadecylthio-2',3'-diethoxychalcone, 4-nonadecylthio-2',4'-diethoxychalcone, 4-nonadecylthio-2',5'-diethoxychalcone, 4-nonadecylthio-2',6'-diethoxychalcone, 4-nonadecylthio-2',3',4'-triethoxychalcone, 4-nonadecylthio-2',3',5'-triethoxychalcone, 4-nonadecylthio-2',4',5'-triethoxychalcone, 4-eicosylthio-2'-butoxychalcone, 4-eicosylthio-4'-butoxychalcone, 4-eicosylthio-3',4'-dibutoxychalcone, 4-heneicosylthio-3',5'-dibutoxychalcone, 4-heneicosylthio-3',4',5'-tributoxychalcone, 4-heneicosylthio-4'-heptyloxychalcone, 4-heneicosylthio-3',4',5'-tripentyloxychalcone, 4-docosylthio-4'-hexyloxychalcone, 4-docosylthio-3',4'-dihexyloxychalcone, 4-docosylthio-3',5'-dihexyloxychalcone, 4-docosylthio-2'-heptyloxychalcone, 4-docosylthio-4'-heptyloxychalcone, 4-docosylthio-3',4'-diheptyloxychalcone, 4-docosylthio-3',4',5'-triheptyloxychalcone, 4-isobutylthio-4'-octyloxychalcone, 4-isobutylthio-3',4',5'-trioctyloxychalcone, 4-isobutylthio-4'-nonyloxychalcone, 4-isobutylthio-3',4'-dinonyloxychalcone, 4-t-butylthio-3',4',5'-trinonyloxychalcone, 4-t-butylthio-2′-decyloxychalcone, 4-t-butylthio-4′-decyloxychalcone, 4-t-butylthio-3′,4′-didecyloxychalcone, 4-t-butylthio-3′,4′,5′-tridecyloxychalcane, 4-t-butylthio-3′ -dodecyloxychalcone, 4-isohexylthio-4′-dodecyloxychalcone, 4-isohexylthio-4′-tridecyloxychalcone, 4-isohexylthio-3′,5′-di(tridecyloxy)chalcone, 4-isoheptylthio-4′-tetradecyloxychalcone, 4-isoheptylthio-3′,4′-di(pentadecyloxy)chalcone, 4-isooctylthio-3′,5′-di-(pentadecyloxy)chalcone, 4-isooctylthio-4′-hexadecyloxychalcone, 4-isooctylthio-3′,4′,5′-tri(hexadecyl)-chalcone, 4-isooctylthio-3′-heptadecyloxychalcone, 4-butylthio-4′-heptadecyloxychalcone, 4-butylthio-3′,5′-di(heptadecyloxy)chalcone, 3,4-bis(butylthio)-3′-octadecyloxychalcone, 3,4,5-tris(butylthio)-4′-octadecyloxychalcone, 4-butylthio-3′,4′-di(octadecyloxy)chalcone, 4-butylthio-3′,4′,5′-tri(octadecyloxy)chalcone, 3,4,5-tris(butylthio)-4′-nonadecyloxychalcone, 4-butylthio-3′,4′-di(nonadecyloxy)chalcone, 3,4,5-tris(hexylthio)-4′-eicosyloxychalcone, 4-butylthio-3′,4-di(eicosyloxy)chalcone, 4-butylthio-4′-heneicosyloxychalcone, 4-butylthio-3′,5′-di(heneicosyloxy)chalcone, 4-ethylthio-3′-chlorochalcone, 4-ethylthio-4′-bromochalcone, 4-ethylthio-4′-iodochalcone, 4-octylthio-3′-chlorochalcone, 3-octylthio-4′-fluorochalcone, 3-octylthio-4′-iodochalcone, 3-octylthio-4′-dimethylaminochalcone, 3-octylthio-4′-aminochalcone, 3-octylthio-4′-nitrochalcone, or 3-octylthio-3′-chlorochalcone.

Benzaldehyde and preferred benzaldehyde derivatives employed in the chalcone derivative compounds represented by the general formula (II) according to the present invention may preferably include, for example 3-chlorobenzaldehyde, 2,5-dichlorobenzaldehyde, 2,4,6-trichlorobenzaldehyde, 2,3,4,5-tetrachlorobenzaldehyde, 3-fluorobenzaldehyde, 4-fluorobenzaldehyde, 3,4-difluorobenzaldehyde, 3,5-difluorobenzaldehyde, 2,4-difluorobenzaldehyde, 2,6-difluorobenzaldehyde, 2,3,4-trifluorobenzaldehyde, 2,4,6-trifluorobenzaldehyde, 2,3,5-trifluorobenzaldehyde, 2,3,4,6-tetrafluorobenzaldehyde, 2,3,5,6-tetrafluorobenzaldehyde, 2,3,4,5,6-pentafluorobenzaldehyde, 3-bromobenzaldehyde, 4-bromobenzaldehyde, 2,5-dibromobenzaldehyde, 3,4-dibromobenzaldehyde, 2,3,4-tribromobenzaldehyde, 2,4,6-tribromobenzaldehyde, 2,3,4,5-pentabromobenzaldehyde, 3-iodobenzaldehyde, 4-iodobenzaldehyde, 2,3-diiodobenzaldehyde, 3,5-diiodobenzaldehyde, 2,3,4-triiodobenzaldehyde, 2,4,6-triiodobenzaldehyde, 3-hydroxybenzaldehyde, 4-hydroxybenzaldehyde, 2,3-dihydroxybenzaldehyde, 2,4-dihydroxybenzaldehyde, 2,6-dihydroxybenzaldehyde, 3,4-dihydroxybenzaldehyde, 2,3,6-trihydroxybenzaldehyde, 2,4,5-trihydroxybenzaldehyde, 2,3,4,5-tetrahydroxybenzaldehyde, 2,3,4,6-tetrahydroxybenzaldehyde, 3-methylbenzaldehyde, 2,3-dimethylbenzaldehyde, 2,6-dimethylbenzaldehyde, 3,4-dimethylbenzaldehyde, 2,3,4-trimethylbenzaldehyde, 2,3,5-trimethyl benzaldehyde, 2,4,6-trimethylbenzaldehyde, 3-ethylbenzaldehyde, 4-ethylbenzaldehyde, 2,4- diethylbenzaldehyde, 2,5-diethylbenzaldehyde, 3,5-diethylbenzaldehyde, 2,3,5-triethylbenzaldehyde, 3,4,5-triethylbenzaldehyde, 2-propylbenzaldehyde, 3-propylbenzaldehyde, 2,4-dipropylbenzaldehyde, 3,4,5 tripropylbenzaldehyde, 2-butylbenzaldehyde, 3-butylbenzaldehyde, 4-butylbenzaldehyde, 3,4-dibutylbenzaldehyde, 3-pentylbenzaldehyde, 4-pentylbenzaldehyde, 4-hexylbenzaldehyde, 2-aminobenzaldehyde, 3-aminobenzaldehyde, 3,5-diaminobenzaldehyde, 2-dimethylaminobenzaldehyde, 3-nitrobenzaldehyde, 3,5-dinitrobenzaldehyde, 4-phenylbenzaldehyde, 3-acetylbenzaldehyde, 4-acetylbenzaldehyde, 3,5-diacetylbenzaldehyde, 3-methoxybenzaldehyde, 2,3-dimethoxybenzaldehyde, 2,5-dimethoxybenzaldehyde, 2,6-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,5-trimethoxybenzaldehyde, 2,4,6-trimethoxybenzaldehyde, 3,4,5-trimethoxybenzaldehyde, 2,3,4,6-tetramethoxybenzaldehyde, 4-ethoxybenzaldehyde, 2,6-diethoxybenzaldehyde, 2,3,5-triethoxybenzaldehyde, 4-propyloxybenzaldehyde, 3,4-dipropyloxybenzaldehyde, 3,4,5-tripropyloxybenzaldehyde, 2-butoxybenzaldehyde, 3-butoxybenzaldehyde, 4-butoxybenzaldehyde, 3,5-dibutoxybenzaldehyde, 3,4,5-tributoxybenzaldehyde, 3-pentyloxybenzaldehyde, 4-pentyloxybenzaldehyde, 3,4,5-tripentyloxybenzaldehyde, 2-hexyloxybenzaldehyde, 4-hexyloxybenzaldehyde, 3,5-dihexyloxybenzaldehyde, 3,4,5-trihexyloxybenzaldehyde, 4-heptyloxybenzaldehyde, 3,5-diheptyloxybenzaldehyde, 3,4,5-triheptyloxybenzaldehyde, 2-octyloxybenzaldehyde, 3-octyloxybenzaldehyde, 4-octyloxybenzaldehyde, 3,4-dioctyloxybenzaldehyde, 2-nonyloxybenzaldehyde, 4-nonyloxybenzaldehyde, 3,4,5-trinonyloxybenzaldehyde, 4-decyloxybenzaldehyde, 3,4-didecyloxybenzaldehyde, 4-dodecyloxybenzaldehyde, 3,5-didodecyloxybenzaldehyde, 4-tridecyloxybenzaldehyde, 3,4-di(tridecyloxy)benzaldehyde, 3,5-di(tridecyloxy)benzaldehyde, 4-tetradecyloxybenzaldehyde, 3,4-di(tetradecyloxy)benzaldehyde, 3,5-di(tetradecyloxy)benzaldehyde, 2-pentadecyloxybenzaldehyde, 3-pentadecyloxybenzaldehyde, 4-pentadecyloxybenzaldehyde, 3,4,5-tri-(pentadecyloxy)benzaldehyde, 4-hexadecyloxybenzaldehyde, 3,4-di(hexadecyloxy)benzaldehyde, 3,4,5-tri-(hexadecyloxy)benzaldehyde, 3-heptadecyloxybenzaldehyde, 4-heptadecyloxybenzaldehyde, 3,5-di-(heptadecyloxy)benzaldehyde, 4-octadecyloxybenzaldehyde, 3,5-di(octadecyloxy)benzaldehyde, 3,4,5-tri-(octadecyloxy)benzaldehyde, 4-nonadecyloxybenzaldehyde, 3,4-di(nonadecyloxy)benzaldehyde, 3-eicosyloxybenzaldehyde, 4-eicosyloxybenzaldehyde, 3-heneicosyloxybenzaldehyde, 4-heneicosyloxybenzaldehyde, 3-docosyloxybenzaldehyde or 4-docosyl oxybenzaldehyde.

The chalcone derivative compounds of the above formula (II), obtained in accordance with the present invention, may preferably include, for example 3-chloro-4′-methylthiochalcone, 2,5-dichloro-4′-methylthiochalcone, 2,4,6-trichloro-4′-methylthiochalcone, 2,3,4,5-tetrachloro-4′-methylthiochalcone, 3-fluoro-4′-methylthiochalcone, 4-fluoro-4′-methylthiochalcone, 3,4-difluoro-4′-methylthiochalcone, 2,5-difluoro-4′-methylthiochalcone, 3,5-difluoro-4′-methylthiochalcone, 2,4-difluoro-4′-methylthiochalcone, 2,6-difluoro-4′-methylthiochalcone, 2,3,4-trifluoro-4′-methylthiochalcone, 3,4,5-trifluoro-4′-methylthiochalcone, 2,4,6-trifluoro-4′-methylthiochalcone, 2,3,5-trifluoro-4′-methylthiochalcone, 2,3,4,6-tetrafluoro-4′-methylthiochalcone, 2,3,5,6-tetrafluoro-4′-methylthiochalcone, 2,3,4,5,6-pentafluoro-4′-methylthiochalcone, 3-bromo-4′-methylthiochalcone, 4-bromo-4′-methylthiochalcone, 2,4-dibromo-4′-methylthiochalcone, 2,5-dibromo-4′-methylthiochalcone, 3,4-dibromo-4′-methylthiochalcone, 2,3,4-tribromo-4′-methylthiochalcone, 2,4,6-tribromo-4′- methylthiochalcone, 2,3,4,5-pentabromo-4′-methylthiochalcone, 3-iodo-4′-methylthiochalcone, 4-iodo-4′-methylthiochalcone, 2,3-diiodo-4′-methylthiochalcone, 3,5-diiodo-4′-methylthiochalcone, 2,3,4-triiodo-4′-methylthiochalcone, 2,4,6-triiodo-4′-methylthiochalcone, 3-hydroxy-4′-methylthiochalcone, 4-hydroxy-4′-methylthiochalcone, 2,3-dihydroxy-4′-methylthiochalcone, 2,4-dihydroxy-4′-methylthiochalcone, 2,6-dihydroxy-4′-methylthiochalcone, 3,4-dihydroxy-4′-methylthiochalcone, 2,3,6-trihydroxy-4′-methylthiochalcone, 2,4,6-trihydroxy-4′-methylthiochalcone, 2,3,4,5-tetrahydroxy-4′-methylthiochalcone, 2,3,4,6-tetrahydroxy-4′-methylthiochalcone, 3-methyl-4′-methylthiochalcone, 2,3-dimethyl-4′-ethylthiochalcone, 2,5-dimethyl-4′-ethylthiochalcone, 2,6-dimethyl-4′-ethylthiochalcone, 3,4-dimethyl-4′-ethylthiochalcone, 2,3,4-trimethyl-4′-ethylthiochalcone, 2,3,5-trimethyl-4′-ethylthiochalcone, 2,4,6-trimethyl-4′-ethylthiochalcone, 2,3,4,5-tetramethyl-4′-ethylthiochalcone, 2-ethyl-4′-ethylthiochalcone, 3-ethyl-4′-ethylthiochalcone, 4-ethyl-4′-ethylthiochalcone, 2,4-diethyl-4′-ethylthiochalcone, 2,5-diethyl-4′-ethylthiochalcone, 3,5-diethyl-4′-ethylthiochalcone, 2,3,5-triethyl-4′-ethylthiochalcone, 3,4,5-triethyl-4′-ethylthiochalcone, 2-propyl-4′-ethylthiochalcone, 3-propyl-4′-ethylthiochalcone, 2,4-dipropyl-4′-ethylthiochalcone, 3,5-dipropyl-4′-ethylthiochalcone, 3,4,5-tripropyl-4′-ethylthiochalcone, 2-butyl-4′-ethylthiochalcone, 3-butyl-4′-ethylthiochalcone, 4-butyl-4′-ethylthiochalcone, 3,4-dibutyl-4′-ethyl thiochalcone, 2-pentyl-4′-ethylthiochalcone, 3-pentyl-4′-ethylthiochalcone, 4-pentyl-4′-ethylthiochalcone, 2-hexyl-4′-ethylthiochalcone, 3-hexyl-4′-ethylthiochalcone, 4-hexyl-4′-ethylthiochalcone, 2-amino-4′-ethylthiochalcone, 3-amino-4′-ethylthiochalcone, 3,4-diamino-4′-ethylthiochalcone, 3,5-diamino-4′-ethylthiochalcone, 2-dimethylamino-4′-ethylthiochalcone, 3-dimethylamino-4′-ethylthiochalcone, 3-nitro-4′-ethylthiochalcone, 3,5-dinitro-4′-ethylthiochalcone, 2-cyano-4′-ethylthiochalcone, 3-cyano-4′-ethylthiochalcone, 4-cyano-4′-ethylthiochalcone, 3-phenyl-4′-ethylthiochalcone, 4-phenyl-4′-ethylthiochalcone, 3-acetyl-4′-ethylthiochalcone, 4-acetyl-4′-ethylthiochalcone, 3-methoxy-4′-ethylthiochalcone, 2,3-dimethoxy-4′-ethylthiochalcone, 2,5-dimethoxy-4′-ethylthiochalcone, 2,6-dimethoxy-4′-ethylthiochalcone, 3,5-dimethoxy-4′-ethylthiochalcone, 2,3,4-trimethoxy-4′-ethylthiochalcone, 2,3,5-trimethoxy-4′-ethylthiochalcone, 2,3,6-trimethoxy-4′-ethylthiochalcone, 2,4,5-trimethoxy-4′-ethylthiochalcone, 2,4,6-trimethoxy-4′-ethylthiochalcone, 3,4,5-trimethoxy-4′-ethylthiochalcone, 2,3,4,6-tetramethoxy-4′-ethylthiochalcone, 4-ethoxy-4′-propylthiochalcone, 2,6-diethoxy-4′-propylthiochalcone, 2,3,5-triethoxy-4′-propylthiochalcone, 4-propyloxy-4′-propylthiochalcone, 3,4-dipropyloxy-4′-propylthiochalcone, 3,4,5-tripropyloxy-4′-propylthiochalcone, 2-butoxy-4′-propylthiochalcone, 3-butoxy-4′-propylthiochalcone, 4-butoxy-4′-propylthiochalcone, 3,5-dibutoxy-4′-propylthiochalcone, 3,4,5-tributoxy-4′-propylthiochalcone, 3-pentyloxy-4′-propylthiochalcone, 4-pentyloxy-4′-propylthiochalcone, 3,4,5-tripentyloxy-4′-propylthiochalcone, 2-hexyloxy-4′-propylthiochalcone, 4-hexyloxy-4′-propylthiochalcone, 3,5-dihexyloxy-4′-propylthiochalcone, 3,4,5-trihexyloxy-4′-propylthiochalcone, 4-heptyloxy-4′-propylthiochalcone, 3,5-diheptyloxy-4′-propylthiochalcone, 3,4,5-triheptyloxy-4′-propylthiochalcone, 2-octyloxy-4′-propylthiochalcone, 3-octyloxy-4′-propylthiochalcone, 4-octyloxy-4′-propylthiochalcone, 3,4-dioctyloxy-4′-propylthiochalcone, 2-nonyloxy-4′-propylthiochalcone, 3-nonyloxy-4′-propylthiochalcone, 4-nonyloxy-4′-propylthiochalcone, 3,4,5-trinonyloxy-4′-propylthiochalcone, 3-decyloxy-4′-propylthiochalcone, 4-decyloxy-4′-propylthiochalcone, 3,4-didecyloxy-4′-propylthiochalcone, 3,5-didecyloxy-4′-propylthiochalcone, 2-dodecyloxy-4′-propylthiochalcone, 3-dodecyloxy-4′-propylthiochalcone, 4-dodecyloxy-4′-propylthiochalcone, 4-tridecyloxy-4′-propylthiochalcone, 3,4-di(tridecyloxy)-4′-propylthiochalcone, 3,5-di(tridecyloxy)-4′-propylthiochalcone, 4-tetradecyloxy-4′-propylthiochalcone, 3,4-di(tetradecyloxy)-4′-propylthiochalcone, 3,5-di(tetradecyloxy)-4′-propylthiochalcone, 2-pentadecyloxy-4′-propylthiochalcone, 4-pentadecyloxy-4′-propylthiochalcone, 3,4,5-tri(pentadecyloxy)-4′-propylthiochalcone, 4-hexadecyloxy-4′-propylthiochalcone, 3,4-di(hexadecyloxy)-4′-propylthiochalcone, 3,4,5-tri(hexadecyloxy)-4′-propylthiochalcone, 3-heptadecyloxy-4′-propylthiochalcone, 4-heptadecyloxy-4′-propylthiochalcone, 3,5-di(heptadecyloxy)-4′-propylthiochalcone, 4-octadecyloxy-4′-propylthiochalcone, 3,5-di(octadecyloxy)-4′-propylthiochalcone, 3,4,5-tri(octadecyloxy)-4′-propylthiochalcone, 4-nonadecyloxy-4′-propylthiochalcone, 3,4-di(nonadecyloxy)-4′-propylthiochalcone, 3-eicosyloxy-4′-propylthiochalcone, 4-eicosyloxy-4′-propylthiochalcone, 3,4-di(eicosyloxy)-4′-propylthiochalcone, 3-heneicosyloxy-4′-propylthiochalcone, 4-heneicosyloxy-4′-propylthiochalcone, 3-docosyloxy-4′-propylthiochalcone, 4-docosyloxy-4′-propylthiochalcone, 4′-butylthiochalcone, 2-chloro-4′-butylthiochalcone, 3-chloro-4′-butylthiochalcone, 4-chloro-4′-butylthiochalcone, 2,5-dichloro-4′-butylthiochalcone, 2,4,6-trichloro-4′-butylthiochalcone, 2,3,4,5-tetrachloro-4′-pentylthiochalcone, 3-fluoro-4′-pentylthiochalcone, 4-fluoro-4′-pentylthiochalcone, 3,4-difluoro-4′-pentylthiochalcone, 3,5-difluoro-4′-pentylthiochalcone, 2,4-difluoro-4′-pentylthiochalcone, 2,6-difluoro-4′-hexylthiochalcone, 2,3,4-trifluoro-4′-hexylthiochalcone, 2,4,6-trifluoro-4′-hexylthiochalcone, 2,3,5-trifluoro-4′-hexylthiochalcone, 2,3,4,6-tetrafluoro-4′-hexylthiochalcone, 2,3,5,6-tetrafluoro-4′-hexylthiochalcone, 2,3,5,6-tetrafluoro-4′-hexylthiochalcone, 2,3,4,5,6-pentafluoro-4′-hexylthiochalcone, 3-bromo-4′-heptylthiochalcone, 4-bromo-4′-heptylthiochalcone, 2,5-dibromo-4′-heptylthiochalcone, 3,4-dibromo-4′-heptylthiochalcone, 2,3,4-tribromo-4′-heptylthiochalcone, 2,4,6-tribromo-4′-heptylthiochalcone, 2,3,4,5-tetrabromo-4′-heptylthiochalcone, 3-iodo-4′-octylthiochalcone, 4-iodo-4′-octylthiochalcone, 2,3-diiodo-4′-octylthiochalcone, 3,5-diiodo-4′-octylthiochalcone, 2,3,4-triiodo-4′-octylthiochalcone, 2,4,6-triiodo-4′-octylthiochalcone, 3-hydroxy-4′-decylthiochalcone, 4-hydroxy-4′-decylthiochalcone, 2,3-dihydroxy-4′-decylthiochalcone, 2,4-dihydroxy-4′-decylthiochalcone, 2,6-dihydroxy-4′-decylthiochalcone, 3,4-dihydroxy-4′-decylthiochalcone, 2,3,6-trihydroxy-4′-decylthiochalcone, 2,4,5-trihydroxy-4′-undecylthiochalcone, 2,3,4,5-tetrahydroxy-4′-undecylthiochalcone, 2,3,4,6-tetra hydroxy-4′-undecylthiochalcone, 3-methyl-4′-undecylthiochalcone, 2,3-dimethyl-4′-undecylthiochalcone, 2,6-dimethyl-4′-dodecylthiochalcone, 3,4-dimethyl-4′-dodecylthiochalcone, 2,3,4-trimethyl-4′-dodecylthiochalcone, 2,3,5-trimethyl-4′-dodecylthiochalcone, 2,4,6-trimethyl-4′-dodecylthiochalcone, 3-ethyl-4′-tridecylthiochalcone, 4-ethyl-4′-tridecylthiochalcone, 2,4-diethyl-4′-tridecylthiochalcone, 2,5-diethyl-4′-tridecylthiochalcone, 3,5-diethyl-4′-tridecylthiochalcone, 2,3,5-triethyl-4′-tridecylthiochalcone, 3,4,5-triethyl-4′-tridecylthiochalcone, 2-propyl-4′-tetradecylthiochalcone, 3-propyl-4′-tetradecylthiochalcone, 2,4-dipropyl-4′-tetradecylthiochalcone, 3,4,5-tripropyl-4′-tetradecylthiochalcone, 2-butyl-4′-tetradecylthiochalcone, 3-butyl-4′-tetradecylthiochalcone, 4-butyl-4′-pentadecylthiochalcone, 3,4-dibutyl-4′-pentadecylthiochalcone, 3-pentyl-4′-pentadecylthiochalcone, 4-pentyl-4′-pentadecylthiochalcone, 4-hexyl-4′-pentadecylthiochalcone, 2-amino-4′-pentadecylthiochalcone, 3-amino-4′-pentadecylthiochalcone, 3,5-diamino-4′-hexadecylthiochalcone, 2-dimethylamino-4′-hexadecylthiochalcone, 3-nitro-4′-hexadecylthiochalcone, 3,5-dinitro-4′-hexadecylthiochalcone, 4-phenyl-4′-hexadecylthiochalcone, 3-acetyl-4′-heptadecylthiochalcone, 4-acetyl-4′-heptadecylthiochalcone, 3,5-diacetyl-4′-heptadecylthiochalcone, 3-methoxy-4′-heptadecylthiochalcone, 2,3-dimethoxy-4′-heptadecylthiochalcone, 2,5-dimethoxy-4′-heptadecylthiochalcone, 2,6-dimethoxy-4′-heptadecylthiochalcone, 3,5 dimethoxy-4′-heptadecylthiochalcone, 2,3,4-trimethoxy-4′-octadecylthiochalcone, 2,3,5-trimethoxy-4′-octadecyclthiochalcone, 2,3,6-trimethoxy-4′-octadecylthiochalcone, 2,4,5-trimethoxy-4′-octadecylthiochalcone, 2,4,6-trimethoxy-4′-octadecylthiochalcone, 3,4,5-trimethoxy-4′-octadecylthiochalcone, 2,3,4,6-tetramethoxy-4′-octadecylthiochalcone, 4-ethoxy-4′-nonadecylthiochalcone, 2,6-diethoxy-4′-nonadecylthiochalcone, 2,3,5-triethoxy-4′-nonadecylthiochalcone, 4-propyloxy-4′-eicosylthiochalcone, 3,4-dipropyloxy-4′-eicosylthiochalcone, 3,4,5-tripropyloxy-4′-eicosylthiochalcone, 2-butoxy-4′-eicosylthiochalcone, 3-buthoxy-4′-eicosylthiochalcone, 4-butoxy-4′-eicosylthiochalcone, 3,5-dibutoxy-4′-heneicosylthiochalcone, 3,4,5-tributoxy-4′-heneicosylthiochalcone, 3-pentyloxy-4′-heneicosylthiochalcone, 4-pentyloxy-4′-heneicosylthiochalcone, 3,4,5-tripentyloxy-4′-heneicosylthiochalcone, 2-hexyloxy-4′-heneicosylthiochalcone, 4-hexyloxy-4′-docosylthiochalcone, 3,5-dihexyloxy-4′-docosylthiochalcone, 3,4,5-trihexyloxy-4′-docosylthiochalcone, 4-heptyloxy-4′-docosylthiochalcone, 3,5-diheptyloxy-4′-docosylthiochalcone, 3,4,5-triheptyloxy-4′-docosylthiochalcone, 2-octyl oxy-4′-isobutylthiochalcone, 3-octyloxy-4′-isobutylthiochalcone, 4-octyloxy-4′-isobutylthiochalcone, 3,4-dioctyloxy-4′-isobutylthiochalcone, 2-nonyloxy-4′-isobutylthiochalcone, 4-nonyloxy-4′-isobutylthiochalcone, 3,4,5-trinonyloxy-4′-t-butylthiochalcone, 4-decyloxy-4′-t-butylthiochalcone, 3,4-didecyloxy-4′-t-butylthiochalcone, 4 dodecyloxy-4′-isohexylthiochalcone, 3,5-didocyloxy-4′-isohexylthiochalcone, 4-tridecyloxy-4′-isohexylthiochalcone, 3,4-di(tridecyloxy)-4′-isohexylthiochalcone, 3,5-di(tridecyloxy)-4′-isohexylthiochalcone, 4-tetradecyloxy-4′-isoheptylthiochalcone, 3,4-di(tetradecyloxy)-4′-isoheptylthiochalcone, 3,5-di-(tetradecyloxy)-4′-isoheptylthiochalcone, 2-pentadecyloxy-4′-isoheptylthiochalcone, 4-pentadecyloxy-4′-isoheptylthiochalcone, 3,4,5-tri(pentadecyloxy)-4′-isooctylthiochalcone, 4-hexadecyloxy-4′-isooctylthiochalcone, 3,4-di(hexadecyloxy)-4′-isooctylthiochalcone, 3,4,5-tri(hexadecyloxy)-4′-isooctylthiochalcone, 3-heptadecyloxy-4′-isooctylthiochalcone, 4-heptadecyloxy-4′-butylthiochalcone, 3,5-di(heptadecyloxy)-4′-butylthiochalcone, 4-octadecyloxy-3′,4′,5′-tris(butylthio)chalcone, 3,5-di(octadecyloxy)-4′-butylthiochalcone, 3,4,5-tri(octadecyloxy)-4′-butylthiochalcone, 4-nonadecyloxy-3′,4′,5′-tris(hexylthio)chalcone, 3,4-di(nonadecyloxy)-4′-butylthiochalcone, 3-eicosyloxy-3′,4′-bis(octylthio)chalcone, 4-eicosyloxy-3′,4′,5′-tris(octylthio)chalcone, 3-heneicosyloxy-4′-butylthiochalcone, 4-heneicosyloxy-4′-butylthiochalcone, 3-docosyloxy-4′-butylthiochalcone, 4-docosyloxy-4′-butylthiochalcone or 4-octadecyloxy-4′-methylthiochalcone.

As described hereinabove, the chalcone derivative compounds of the present invention represented by the formulae (I) and (II) can be used in a number of usages including pharmaceuticals, agricultural chemicals, anti-bacterial substances, insecticides or repellents.

Examples

Although with reference to Examples, the present invention will be explained further in detail hereinbelow, the invention is not limited thereby.

Example 1

1.52 g (0.01 mol) of 4-methylthiobenzaldehyde and 1.20 g (0.01 mol) of acetophenone were charged in a reaction vessel with 20 ml of ethanol and stirred at 10 to 25° C. Into this reaction vessel was charged dropwise a mixed solution of 1 g of a 40% aqueous solution of sodium hydroxide and 10 ml of ethanol. After termination of dropwise charging, the reaction was carried out for 30 minutes while the temperature was maintained at 10 to 25° C. Then, 20 ml of a 0.5 N aqueous solution of hydrochloric acid was added to the reaction system and stirred to terminate the reaction. A separated solid was obtained by filtration and the so-produced solid was washed with water several times and dried.

This crude product was recrystallized with an ethanol solvent to give 1.48 g of a purified product of 4-methylthiochalcone with the yield rate of 58 %. The melting point of the product was 83° C. The IR and $^1H$-NMR charts are shown in Figs.1 and 2, respectively. The results of the IR, $^1H$-NMR and mass spectrometry are as given below.

IR (NaCl / $CCl_4$)
1670 (C = O) ; 1330 (S - $CH_3$),
1490, 1590, 1610 (aryl)$cm^{-1}$
$^1H$ - NMR ($CDCl_3$)
$\delta$ 2.5 (S, 3H, ArS - $CH_3$),
7.2 - 8.2 (dd, 4H, aryl ; m,
4H, aryl, S, 2H, alkene)ppm.

| Mass Spectrometry (m/Z) | | |
|---|---|---|
| $M^+$ | (506) | 100.0 |
| M + 1 | (507) | 38.9 |
| M + 2 | (508) | 8.0 |
| M + 3 | (509) | 1.1 |

Example 2

1.52 g (0.01 mol) of 4-methylthiobenzaldehyde and 1.55 g (0.01 mol) of 4′-chloroacetophenone were charged into a reaction vessel with 30 ml of ethanol and the reaction was carried out for one hour while the temperature was maintained at 30° to 40° C. Then, 20 ml of a 0.5 N aqueous solution of hydrochloric acid was added to the reaction system and stirred to terminate the reaction. A separated solid was obtained by filtration. The so-produced solid was washed several times with water.

This crude product was recrystallized with an ethanol solvent to produce 1.30 g of a purified product of 4-methylthio-4′-chlorochalcone with a yield rate of 45 %. The melting point of the product was 149° C. The results of the IR, $^1H$-NMR and mass spectrometry are as shown below.

IR (NaCl / $CCl_4$)
1670 (C = O) ; 1330 (S - $CH_3$),
1490, 1590, 1600 (aryl)$cm^{-1}$
$^1H$ - NMR ($CDCl_3$)
$\delta$ 2.5 (S, 3H, ArS - $CH_3$),
7.4 - 8.1(dd, 4H, aryl ; dd,
4H, aryl, S, 2H, alkene)ppm.

| Mass Spectrometry (m/Z) | | |
|---|---|---|
| $M^+$ | (288) | 100.0 |
| M + 1 | (289) | 19.0 |
| M + 2 | (290) | 39.0 |
| M + 3 | (291) | 7.3 |

Example 3

1.52 g (0.01 mol) of 4-methylthiobenzaldehyde and 1.99 g (0.01 mol) of 4′-bromoacetophenone were charged in accordance with Example 2, and the reaction and the purification were also performed in accordance with Example 2 to produce 1.40 g of a purified product of 4-methylthio-4′-bromochalcone with a yield rate of 42%. The melting point of the product was 161 ° C. The results of the IR, $^1$H-NMR and mass spectrometry are as shown below.
IR (NaCl / $CCl_4$)
1665 (C = O) ; 1330 (S - $CH_3$),
1490, 1590, 1600 (aryl)cm$^{-1}$
$^1$H - NMR ($CDCl_3$)
δ 2.5 (S, 3H, ArS-$CH_3$),
7.2 - 8.0 (dd, 4H, aryl ; dd,
4H, aryl, S, 2H, alkene)ppm.

| Mass Spectrometry (m/Z) | | |
|---|---|---|
| $M^+$ | (332) | 100.0 |
| M + 1 | (333) | 19.1 |
| M + 2 | (334) | 104.0 |
| M + 3 | (335) | 19.5 |
| M + 4 | (336) | 6.3 |

Example 4

1.52 g (0.01 mol) of 4-methylthiobenzaldehyde and 1.36 g (0.01 mol) of 4′-hydroxyacetophenone were charged in accordance with Example 2 and the reaction and purification were similarly performed in accordance with Example 2 to produce 0.64 g of a purified product of 4-methylthio-4′-hydroxychalcone with a yield rate of 24 %. The melting point of the product was 171 ° C. The following are the results of the IR, $^1$H-NMR and mass spectrometry.
IR (KBr Tablet)
1640 (C = O) ; 1345 (S - $CH_3$),
1490, 1585, 1600 (aryl)cm$^{-1}$
$^1$H - NMR (MeOH - $d_4$)
δ 2.5 (S, 3H, S - $CH_3$),
4.85 (S, 1H, Ar - OH),
6.8 - 8.2 (dd, 4H, aryl ; dd,
4H, aryl),
7.7 (S, 2H, alkene)ppm.

| Mass Spectrometry (m/Z) | | |
|---|---|---|
| $M^+$ | (284) | 100.0 |
| M + 1 | (285) | 19.3 |
| M + 2 | (286) | 6.5 |
| M + 3 | (287) | 1.1 |

Example 5

1.52 g (0.01 mol) of 4-methylthiobenzaldehyde and 1.35 g (0.01 mol) of 4′-aminoacetophenone were charged with 30 ml of ethanol into a reaction vessel and stirred at 20° to 30° C. A mixed solution of 1 g of a 40% aqueous solution of sodium hydroxide and 10 ml of ethanol was charged dropwise into the reaction vessel. After termination of dropwise charging, the reaction was carried out for 30 minutes while the reaction temperature was maintained at 10° to 25° C. To the reaction system was then added 50 ml of water and stirred. A separated solid was obtained by filtration. The so-produced solid was washed several times with water and dried.

This crude product was recrystallized with a 1/1 solvent mixture of ethanol/benzene to produce 1.15 g of a purified product of 4-methylthio-4′-aminochalcone with a yield rate of 43 %. The melting point of the product was 168° C. The following are the results of the IR, $^1$H-NMR and mass spectrometry.

IR (KBr Tablet)
1655 (C = O) ; 1340 (S - $CH_3$),
1495, 1580, 1600 (aryl)$cm^{-1}$
$^1$H - NMR ($CDCl_3$),
$\delta$ 2.5 (S, 3H, S - $CH_3$),
7.2 - 8.4 (dd, 4H, aryl ; dd,
4H, aryl ; dd, 2H, alkene)ppm.

| Mass Spectrometry (m/Z) | | |
|---|---|---|
| $M^+$ | (269) | 100.0 |
| M + 1 | (270) | 19.4 |
| M + 2 | (271) | 6.3 |
| M + 3 | (272) | 1.0 |

Example 6

1.52 g (0.01 mol) of 4-methylthiobenzaldehyde and 1.65 g (0.01 mol) of 4′-nitroacetophenone were charged and reacted in accordance with Example 2 and the produced crude product was recrystallized with a 1/1 solvent mixture of ethanol/benzene to produce 1.66 g of 4-methylthio-4′-nitrochalcone with a yield rate of 56 %. The melting point of the product was 159.5° C. The following are the results of the IR, $^1$H-NMR and mass spectrometry.

IR (KBr Tablet)
1655 (C = O) ; 1340 (S - $CH_3$),
1520, 1580 (aryl)$cm^{-1}$
$^1$H - NMR ($CDCl_3$)
$\delta$ 2.5 (S, 3H, S - $CH_3$),
7.3, 8.4 (dd, 4H, aryl),
7.5 - 8.2 (m, 6H, aryl, alkene)ppm.

| Mass Spectrometry (m/Z) | | |
|---|---|---|
| $M^+$ | (299) | 100.0 |
| M + 1 | (300) | 19.3 |
| M + 2 | (301) | 6.9 |
| M + 3 | (302) | 1.0 |

Example 7

1.52 g (0.01 mol) of 4-methylthiobenzaldehyde and 1.63 g (0.01 mol) of 4′-dimethylacetophenone were charged in accordance with Example 5 and the reaction and purification were similarly performed in accordance with Example 5 to produce 1.28 g of a purified product of 4-methylthio-4′-dimethylaminochalcone with a yield rate of 43 %. The melting point of the product was 150.5° C. The following are the results of the IR, $^1$H-NMR and mass spectrometry.
IR ($NaCl / CCl_4$)
1660 (C = O) ; 1360 ($S - CH_3$),
1595, 1650 (aryl)$cm^{-1}$
$^1$H - NMR ($CDCl_3$)
$\delta$ 2.5 (S, 3H, $S - CH_3$).
3.1 (S, 6H, $N - CH_3$),
6.7, 8.0 (dd, 4H, aryl),
7.2, 7.7 (dd, 4H, aryl),
7.5 (S, 2H, alkene)ppm.

| Mass Spectrometry (m/Z) | | |
|---|---|---|
| $M^+$ | (297) | 100.0 |
| M + 1 | (298) | 21.7 |
| M + 2 | (299) | 9.0 |
| M + 3 | (300) | 1.0 |

Example 8

1.52 g (0.01 mol) of 4-methylthiobenzaldehyde and 1.50 g (0.01 mol) of 4′-methoxyacetophenone were charged in accordance with Example 2 and the reaction and purification were similarly performed in accordance with Example 2 to produce 1.18 g of a purified product of 4-methylthio-4′-methoxychalcone with a yield rate of 42 %. The melting point of the product was 112.5° C. The following are the results of the IR, $^1$H-NMR and mass spectrometry.
IR ($NaCl / CCl_4$)
1660 (C = O) ; 1330 ($S - CH_3$),
1590, 1600 (aryl)$cm^{-1}$
$^1$H - NMR ($CDCl_3$)
$\delta$ 2.5 (S, 3H, $S - CH_3$),
3.9 (S, 3H, $O - CH_3$),
7.0, 8.0 (dd, 4H, aryl),
7.3, 7.7 (dd, 4H, aryl),
7.5 (S, 2H, alkene)ppm.

| Mass Spectrometry (m/Z) | | |
|---|---|---|
| $M^+$ | (284) | 100.0 |
| M + 1 | (285) | 20.2 |
| M + 2 | (286) | 6.7 |
| M + 3 | (287) | 1.0 |

Example 9

1.52 g (0.01 mol) of 4-methylthiobenzaldehyde and 1.64 g (0.01 mol) of 4′-ethoxyacetophenone were charged in accordance with Example 2 and the reaction and purification were similarly performed in accordance with Example 2 to produce 1.31 g of a purified product of 4-methylthio-4′-ethoxychalcone with a yield rate of 44 %. The melting point of the product was 107.5° C. The following are the results of the IR, ¹H-NMR and mass spectrometry.

IR (NaCl / $CCl_4$)
1660 (C = O) ; 1330 (S - $CH_3$),
1490, 1590, 1600 (aryl)$cm^{-1}$
¹H - NMR ($CDCl_3$)
δ 1.4 (tri, 3H, $OCH_2\underline{CH_3}$),
2.5 (S, 3H, S - $CH_3$),
4.1 (tetra, 2H, $O\underline{CH_2}CH_3$),
6.9, 8.0 (dd, 4H, aryl),
7.2, 7.6 (dd, 4H, aryl),
7.5, 7.8 (dd, 2H, alkene)ppm.

| Mass Spectrometry (m/Z) | | |
|---|---|---|
| $M^+$ | (298) | 100.0 |
| M + 1 | (299) | 21.3 |
| M + 2 | (300) | 7.0 |
| M + 3 | (301) | 1.1 |

Example 10

1.52 g (0.01 mol) of 4-methylthiobenzaldehyde and 3.88 g (0.01 mol) of 4′-octadecyloxyacetophenone were charged with 40 ml of ethanol into a reaction vessel and the resulting reaction system was stirred at 50° to 60° C and dissolved completely. A mixed solution of 1 g of a 40% aqueous solution of sodium hydroxide and 10 ml of ethanol was charged dropwise into the reaction vessel. After termination of the dropwise addition, the reaction was carried out for eight hours while the temperature was maintained at 30° to 40° C. To the reaction system was then added 20 ml of a 0.5 N aqueous solution of hydrochloric acid and stirred to terminate the reaction. A separated solid was recovered by filtration. The solid thus produced was washed several times with water and dried.

The crude product was recrystallized with an ethanol solvent to produce 4.00 g of a purified product of 4-methylthio-4′-octadecyloxychalcone with a yield rate of 77 %. The melting point of the product was 104° C. The following are the results of the IR, ¹H-NMR and mass spectrometry

IR (NaCl / $CCl_4$)
1660 (C = O) ; 1335 (S - $CH_3$),
1500, 1590, 1600 (aryl),
2850, 2920 (alkane)$cm^{-1}$
¹H - NMR ($CDCl_3$)
δ 0.7 - 1.0 (tri, 3H, $O(CH_2)_{17}\underline{CH_3}$),

1.1 - 2.0 (b, 32H, $OCH_2(\underline{CH_2})_{16}CH_3$),
2.5 (S, 3H, S - $CH_3$),
4.0 (tri, 2H, $O\underline{CH_2}(CH_2)_{16}CH_3$),
7.0, 8.0 (dd, 4H, aryl),
7.3, 7.6 (dd, 4H, aryl),
7.5, 7.8 (dd, 2H, alkene)ppm.

| Mass Spectrometry (m/Z) | | |
|---|---|---|
| $M^+$ | (522) | 100.0 |
| M + 1 | (523) | 39.7 |
| M + 2 | (524) | 12.4 |
| M + 3 | (525) | 2.9 |

Example 11

1.52 g (0.01 mol) of 4-methylthiobenzaldehyde and 1.99 g (0.01 mol) of 3′-bromoacetophenone were charged with 20 ml of ethanol into a reaction vessel and stirred at 20 °C. A mixed solution of 1 g of a 40%-aqueous solution of sodium hydroxide and 10 ml of ethanol was charged dropwise into the reaction vessel. After termination of the dropwise addition, the reaction was carried out for 30 minutes while the temperature was maintained at 20 °C. To the reaction system was then added 20 ml of a 0.5 N aqueous solution of hydrochloric acid and stirred to terminate the reaction. After termination of the reaction, a separated solid was recovered by filtration and washed several times with water and dried.

This crude product was recrystallized with an etyhanol solvent to produce 1.83 g of a purified product of 4-methylthio-3′-bromochalcone with a yield rate of 55 %. The following are the results of IR, $^1$H-NMR and mass spectrometry.
IR (NaCl / $CCl_4$)
1665 (C = O) ; 1330 (S - $CH_3$),
1490, 1590, 1600 (aryl)$cm^{-1}$
$^1$H - NMR ($CDCl_3$)
δ 2.5 (S, 3H, S - $CH_3$),
7.2 - 8.2 (m, 10H, aryl, alkene)ppm.

| Mass Spectrometry (m/Z) | | |
|---|---|---|
| $M^+$ | (322) | 100.0 |
| M + 1 | (333) | 18.9 |
| M + 2 | (334) | 104.0 |
| M + 3 | (335) | 19.5 |
| M + 4 | (336) | 6.3 |
| M + 5 | (337) | 1.0 |

Example 12

1.52 g (0.01 mol) of 4-methylthiobenzaldehyde and 1.34 g (0.01 mol) of 3′-methylacetophenone were charged in accordance with Example 11 and the reaction and purification were similarly performed in accordance with Example 11 to produce 1.93 g of a purified product of 4-methylthio-3′-methylchalcone with a yield rate of 72 %. The following are the results of IR, $^1$H-NMR and mass spectrometry.
IR (NaCl / $CCl_4$)
1660 (C = O) ; 1330 (S - $CH_3$),
1590, 1650 (aryl)$cm^{-1}$
$^1$H - NMR ($CDCl_3$)

δ 2.4 (S, 3H, $C_6H_5$ - $CH_3$),
2.5 (S, 3H, S - $CH_3$),
7.2 - 7.9 (m, 10H, aryl, alkene)ppm.

| Mass Spectrometry (m/Z) | | |
|---|---|---|
| $M^+$ | (268) | 100.0 |
| M + 1 | (269) | 20.0 |
| M + 2 | (270) | 6.3 |
| M + 3 | (271) | 1.2 |

Example 13

1.52 g (0.01 mol) of 4-methylthiobenzaldehyde and 1.50 g (0.01 mol) of 3′-methoxyacetophenone were charged in accordance with Example 11 and the reaction and purification were similarly performed in accordance with Example 11 to produce 2.42 g of a purified product of 4-methylthio-3′-methoxychalcone with a yield rate of 85 %. The results of IR, $^1$H-NMR and mass spectrometry are as shown below.
IR (NaCl / $CCl_4$)
1660 (C = O) ; 1330 (S - $CH_3$),
1590, 1600 (aryl)$cm^{-1}$
$^1$H - NMR ($CDCl_3$)
δ 2.5 (S, 3H, S - $CH_3$),
3.9 (S, 3H, O - $CH_3$),
7.0 - 7.9 (m, 10H, aryl, alkene)ppm.

| Mass Spectrometry (m/Z) | | |
|---|---|---|
| $M^+$ | (284) | 100.0 |
| M + 1 | (285) | 20.2 |
| M + 2 | (286) | 6.7 |
| M + 3 | (287) | 1.0 |

Example 14

1.52 g (0.01 mol) of 4-methylthiobenzaldehyde and 1.35 g (0.01 mol) of 3′-aminoacetophenone were charged with 30 ml of ethanol into a reaction vessel and stirred at 25° C. A mixed solution of 1 g of a 40%-aqueous solution of sodium hydroxide and 10 ml of ethanol was added dropwise into the reaction vessel. After termination of the dropwise addition, the reaction was carried out for 1.5 hour while the temperature was maintained at 25° C. To the reaction system was then added 50 ml of water and stirred. A separated solid was recovered by filtration, washed with water several times and dried.

This crude product was recrystallized with an ethanol solvent to produce 1.04 g of a purified product of 4-mthylthio-3′-aminochalcone with a yield rate of 31 %. The results of IR, $^1$H-NMR and mass spectrometry are as shown below.
IR (NaCl / $CCl_4$)
1660 (C = O) ; 1340 (S - $CH_3$),
1490, 1580, 1600 (aryl)$cm^{-1}$
$^1$H - NMR ($CDCl_3$)
δ 2.5 (S, 3H, S - $CH_3$),
7.2 - 8.4 (m, 10H, aryl, alkene)ppm.

| Mass Spectrometry (m/Z) | | |
|---|---|---|
| $M^+$ | (269) | 100.0 |
| M + 1 | (270) | 19.3 |
| M + 2 | (271) | 6.5 |
| M + 3 | (272) | 1.0 |

Example 15

1.52 g (0.01 mol) of 4-methylthiobenzaldehyde and 1.21 g (0.01 mol) of 3′-dimethylaminoacetophenone were charged in accordance with Example 14 and the reaction and purification were similarly performed in accordance with Example 14 to produce 1.81 g of a purified product of 4-methylthio-3′-dimethylaminochalcone with a yield rate of 61 %. The results of IR, $^1$H-NMR and mass spectrometry are as shown below.
IR (NaCl / $CCl_4$)
1660 (C = O) ; 1350 (S - $CH_3$),
1590, 1650 (aryl)$cm^{-1}$
$^1$H - NMR ($CDCl_3$)
$\delta$ 2.5 (S, 3H, S - $CH_3$),
3.0 (S, 6H, N - $CH_3$),
6.9 - 7.9 (m, 10H, aryl alkene)ppm.

| Mass Spectrometry (m/Z) | | |
|---|---|---|
| $M^+$ | (297) | 100.0 |
| M + 1 | (298) | 21.6 |
| M + 2 | (299) | 6.8 |
| M + 3 | (300) | 1.1 |

Example 16

1.52 g (0.01 mol) of 4-methylthiobenzaldehyde and 1.65 g (0.01 mol) of 3′-nitroacetophenone were charged in accordance with Example 11 and the reaction and purification were similarly performed in accordance with Example 11 to produce 0.68 g of a purified product of 4-methylthio-3′-nitrochalcone with a yield rate of 23 %. The results of IR, $^1$H-NMR and mass spectrometry are as shown below.
IR (NaCl / $CCl_4$)
1655 (C = O) ; 1340 (S - $CH_3$),
1520, 1580 (aryl)$cm^{-1}$
$^1$H - NMR ($CDCl_3$)
$\delta$ 2.5 (S, 3H, S - $CH_3$)
7.2 - 8.8 (m, 10H, aryl, alkene)ppm.

| Mass Spectrometry (m/Z) | | |
|---|---|---|
| $M^+$ | (299) | 100.0 |
| M + 1 | (300) | 19.3 |
| M + 2 | (301) | 6.8 |
| M + 3 | (302) | 1.1 |

The same operation was performed except using 2′-nitroacetophenone in place of 3′-nitroacetophenone to produce 1.54 g of a purified product of 4-methylthio-2′-nitrochalcone with a yield rate of 52 %. The

results of IR, $^1$H-NMR and mass spectrometry are as shown below.
IR (NaCl / $CCl_4$)
1655 (C = O) ; 1340 (S - $CH_3$),
1520, 1580 (aryl)$cm^{-1}$
$^1$H - NMR ($CDCl_3$)
$\delta$ 2.5 (S, 3H, S - $CH_3$),
6.9 - 8.2 (m, 10H, aryl, alkene)ppm.

| Mass Spectrometry (m/Z) | | |
|---|---|---|
| $M^+$ | (299) | 100.0 |
| M + 1 | (300) | 19.2 |
| M + 2 | (301) | 7.0 |
| M + 3 | (302) | 0.9 |

Example 17

1.52 g (0.01 mol) of 4-methylthiobenzaldehyde and 1.80 g (0.01 mol) of 3′,4′-dimethoxyacetophenone were charged in accordance with Example 11 and the reaction and purification were similarly performed in accordance with Example 11 to produce 2.86 g of a purified product of 4-methylthio-3′,4′-dimethoxychalcone with a yield rate of 91 %. The results of IR, $^1$H-NMR and mass spectrometry are as shown below.
IR (NaCl / $CCl_4$)
1660 (C = O) ; 1330 (S - $CH_3$),
1585, 1600 (aryl)$cm^{-1}$
$^1$H - NMR ($CDCl_3$)
$\delta$ 2.5 (S, 3H, S - $CH_3$),
3.9 (S, 3H, O-$CH_3$),
6.8 - 7.9 (m, 9H, aryl, alkene)ppm.

| Mass Spectrometry (m/Z) | | |
|---|---|---|
| $M^+$ | (314) | 100.0 |
| M + 1 | (315) | 21.2 |
| M + 2 | (316) | 7.0 |
| M + 3 | (317) | 1.2 |

Example 18

1.52 g (0.01 mol) of 4-methylthiobenzaldehyde and 2.10 g (0.01 mol) of 3′,4′,5′-trimethoxyacetophenone were charged in accordance with Example 11 and the reaction and purification were similarly performed in accordance with Example 11 to produce 1.70 g of a purified product of 4-methylthio-3′,4′,5′-trimethoxychalcone with a yield rate of 50 %. The results of IR, $^1$H-NMR and mass spectrometry are as shown below.
IR (NaCl / $CCl_4$)
1660 (C = O) ; 1330 (S - $CH_3$)
1580, 1600 (aryl)$cm^{-1}$
$^1$H - NMR ($CDCl_3$)
$\delta$ 2.5 (S, 3H, S - $CH_3$),
3.9 (S, 9H, 0 - $CH_3$),
7.2 - 7.9 (m, 8H, aryl, alkene)ppm.

| Mass Spectrometry (m/Z) | | |
|---|---|---|
| $M^+$ | (344) | 100.0 |
| M + 1 | (345) | 22.4 |
| M + 2 | (346) | 7.6 |
| M + 3 | (347) | 1.2 |

Example 19

1.52 g (0.01 mol) of 4-methylthiobenzaldehyde and 1.96 g (0.01 mol) of 4 acetylbiphenyl were charged in accordance with Example 11 and the reaction and purification were similarly performed in accordance with Example 11 to produce 1.26 g of a purified product of 4-methylthio-4′-phenylchalcone with a yield rate of 38 %. The results of IR, $^1$H-NMR and mass spectrometry are as shown below.
IR (NaCl / $CCl_4$)
1660 (C = O) ; 1330 (S - $CH_3$),
1580, 1600 (aryl)$cm^{-1}$
$^1$H -NMR ($CDCl_3$)
δ 2.5 (S, 3H, S - $CH_3$),
7.2 - 8.2 (m, 14H, aryl, alkene)ppm.

| Mass Spectrometry (m/Z) | | |
|---|---|---|
| $M^+$ | (331) | 100.0 |
| M + 1 | (332) | 25.8 |
| M + 2 | (333) | 8.0 |
| M + 3 | (334) | 1.3 |

Example 20

1.52 g (0.01 mol) of 4-methylthiobenzaldehyde and 1.62 g (0.01 mol) of 4′-acetylacetophenone were charged in accordance with Example 11 and the reaction and purification were similarly performed in accordance with Example 11 to produce 1.80 g of a purified product of 4-methylthio-4′-acetylchalcone with a yield rate of 61 %. The results of IR, $^1$H-NMR and mass spectrometry are as shown below.
IR (NaCl / $CCl_4$)
1655, 1675 (C = O) ; 1340 (S - $CH_3$)
1495, 1585, 1600 (aryl)$cm^{-1}$
$^1$H - NMR ($CDCl_3$)
δ 2.5 (S, 3H, S - $CH_3$),
2.7 (S, 3H, Ph - $COCH_3$),
7.3, 7.6 (dd, 4H, aryl),
7.3, 7.8 (dd, 2H, alkene),
8.1 (S, 4H, aryl)ppm.

| Mass Spectrometry (m/Z) | | |
|---|---|---|
| $M^+$ | (331) | 100.0 |
| M + 1 | (332) | 25.6 |
| M + 2 | (333) | 7.7 |
| M + 3 | (334) | 1.6 |

Example 21

0.50 g (0.003 mol) of 4-ethylthiobenzaldehyde and 0.46 g (0.003 mol) of 3′-chloroacetophenone were charged into a reaction vessel with 20 ml of ethanol and stirred at 20° C. A mixed solution of 1 g of a 40%-aqueous solution of sodium hydroxide and 10 ml of ethanol was added dropwise into the reaction vessel. After termination of the dropwise addition, the reaction was carried out for 30 minutes while the reaction temperature was maintained at 20° C. To the reaction product was added 20 ml of a 0.5 N aqueous solution of hydrochloric acid and stirred to terminate the reaction. A separated solid was recovered by filtration, washed several times with water and dried.

The produced crude product was recrystallized with an ethanol solvent to produce 0.56 g of a purified product of 4-ethylthio-3′-chlorochalcone with a yield rate of 62 %. The melting point of the product was 108.7° C. The results of mass spectrometry and $^1$H-NMR are as shown below.

| Mass Spectrometry (m/Z) | | |
|---|---|---|
| $M^+$ | (302) | 100.0 |
| M + 1 | (303) | 20.0 |
| M + 2 | (304) | 39.3 |
| M + 3 | (305) | 7.5 |
| M + 4 | (306) | 2.5 |

$^1$H - NHR ($CDCl_3$)
δ 1.2 - 1.5 (tri. 3H, $ArSCH_2$ - $CH_3$),
2.9 - 3.2 (tetra, 2H, ArS - $CH_2$ - $CH_3$),
7.2 - 8.0 (m, 10H, aryl, alkene)ppm.

Example 22

0.50 g (0.003 mol) of 4-ethylthiobenzaldehyde and 0.60 g (0.003 mol) of 4′-bromoacetophenone were charged in accordance with Example 21 and the reaction and purification were similarly performed in accordance with Example 21 to produce 0.75 g of a purified product of 4-ethylthio-4′-bromochalcone with a yield rate of 72 %. The melting point of the product was 130.0° C. The results of mass spectrometry and $^1$H-NMR are as shown below.

| Mass Spectrometry (m/Z) | | |
|---|---|---|
| $M^+$ | (346) | 95.7 |
| M + 1 | (347) | 19.0 |
| M + 2 | (348) | 100.0 |
| M + 3 | (349) | 20.1 |
| M + 4 | (350) | 6.5 |

$^1$H - NHR ($CDCl_3$)
δ 1.2 - 1.5 (tri, 3H, $ArSCH_2$ - $CH_3$),
2.9 - 3.2 (tetra, 2H, ArS - $CH_2$ - $CH_3$),
7.2 - 8.0 (m, 10H, aryl, alkene)ppm.

Example 23

0.50 g (0.003 mol) of 4-ethylthiobenzaldehyde and 0.74 g (0.003 mol) of 4′-iodoacetophenone were charged in accordance with Example 21 and the reaction and purification were similarly performed in accordance with Example 21 to produce 0.90 g of a purified product of 4-ethylthio-4′-iodochalcone with a yield rate of 76 %. The melting point of the product was 127.7° C. The results of mass spectrometry and $^1$H-NMR are as shown below.

| Mass Spectrometry (m/Z) | | |
|---|---|---|
| $M^+$ | (394) | 100.0 |
| M + 1 | (395) | 20.0 |
| M + 2 | (396) | 6.7 |
| M + 3 | (397) | 1.1 |

$^1H$ - NHR ($CDCl_3$)
δ 0.8 - 1.1 (tri, 3H, $ArSCH_2CH_2CH_2$ - $CH_3$)
1.3 - 1.8 (m, 4H, $ArSCH_2$ - $CH_2CH_2$ - $CH_3$)
2.9 - 3.2 (tri, 3H, ArS - $CH_2$ - $CH_2CH_2CH_3$)
7.2 - 8.0 (m, 10H, aryl, alkene)ppm.

Example 24

0.49 g (0.0025 mol) of 4-butylthiobenzaldehyde and 0.30 g (0.0025 mol) of acetophenone were charged in accordance with Example 21 and the reaction and purification were similarly performed in accordance with Example 21 to produce 0.41 g of a purified product of 4-butylthiochalcone with a yield rate of 55 %. The melting point of the product was 85.7° C. The results of mass spectrometry and $^1H$-NMR are as shown below.

| Mass Spectrometry (m/Z) | | |
|---|---|---|
| $M^+$ | (296) | 100.0 |
| M + 1 | (297) | 22.5 |
| M + 2 | (298) | 7.3 |
| M + 3 | (299) | 1.3 |

$^1H$ - NHR ($CDCl_3$)
δ 0.8 - 1.1 (tri, 3H, $ArSCH_2CH_2CH_2$ - $CH_3$),
1.3 - 1.8 (m, 4H, $ArSCH_2$ - $CH_2CH_2$ - $CH_3$),
2.9 - 3.2 (tri, 3H, ArS - $CH_2$ - $CH_2CH_2CH_3$),
7.2 - 8.0 (m, 11H, aryl, alkene)ppm.

Example 25

0.49 g (0.0025 mol) of 4-butylthiobenzaldehyde and 0.39 g (0.0025 mol) of 3′-chloroacetophenone were charged in accordance with Example 21 and the reaction and purification were similarly performed in accordance with Example 21 to produce 0.57 g of a purified product of 4-butylthio-3′-chlorochalcone with a yield rate of 69 %. The melting point of the product was 85.0° C. The results of mass spectrometry and $^1H$-NMR are as shown below.

| Mass Spectrometry (m/Z) | | |
|---|---|---|
| $M^+$ | (330) | 100.0 |
| M + 1 | (331) | 22.3 |
| M + 2 | (332) | 38.9 |
| M + 3 | (333) | 8.3 |
| M + 4 | (334) | 2.3 |

$^1H$ - NMR ($CDCl_3$)
δ 0.8 - 1.1 (tri, 3H, $ArSCH_2CH_2CH_2$ - $CH_3$),
1.3 - 1.8 (m, 4H, $ArSCH_2$ - $CH_2CH_2$ - $CH_3$),

2.9 - 3.2 (tri, 3H, ArS - $CH_2$ - $CH_2CH_2CH_3$),
7.2 - 8.0 (m, 10H, aryl, alkene)ppm.

Example 26

0.49 g (0.0025 mol) of 4-butylthiobenzaldehyde and 0.50 g (0.0025 mol) of 4′-bromoacetophenone were charged in accordance with Example 21 and the reaction and purification were similarly performed in accordance with Example 21 to produce 0.63 g of a purified product of 4-butylthio-4′-bromochalcone with a yield rate of 67 %. The melting point of the product was 126.0° C. The results of mass spectrometry and $^1$H-NMR are as shown below.

| Mass Spectrometry (m/Z) | | |
|---|---|---|
| $M^+$ | (374) | 95.5 |
| M + 1 | (375) | 21.5 |
| M + 2 | (376) | 100.0 |
| M + 3 | (377) | 22.5 |
| M + 4 | (378) | 6.7 |
| M + 5 | (379) | 1.1 |

$^1$H - NMR ($CDCl_3$)
$\delta$ 0.8 - 1.1 (tri, 3H, $ArSCH_2CH_2CH_2$ - $CH_3$),
1.3 - 1.8 (m, 4H, $ArSCH_2$ - $CH_2CH_2$ - $CH_3$),
2.9 - 3.2 (tri, 3H, ArS - $CH_2$ - $CH_2CH_2CH_3$),
7.2 - 8.0 (m, 10H, aryl, alkene)ppm.

Example 27

0.49 g (0.0025 mol) of 4-butylthiobenzaldehyde and 0.62 g (0.0025 mol) of 4′-iodoacetophenone were charged in accordance with Example 21 and the reaction and purification were similarly performed in accordance with Example 21 to produce 0.87 g of a purified product of 4-butylthio-4′-iodochalcone with a yield rate of 82 %. Fig.3 shows the $^1$H-NMR chart. The melting point of the product was 142.7° C. The following shows the results of mass spectrometry and $^1$H-NMR.

| Mass Spectrometry (m/Z) | | |
|---|---|---|
| $M^+$ | (422) | 100.0 |
| M + 1 | (423) | 22.3 |
| M + 2 | (424) | 7.1 |
| M + 3 | (425) | 1.3 |

$^1$H - NMR ($CDCl_3$)
$\delta$ 0.8 - 1.1 (tri, 3H, $ArSCH_2CH_2CH_2$ - $CH_3$),
1.3 - 1.8 (m, 4H, $ArSCH_2$ - $CH_2CH_2$ - $CH_3$),
2.9 - 3.2 (tri, 3H, ArS - $CH_2$ - $CH_2CH_2CH_3$),
7.2 - 8.0 (m, 10H, aryl, alkene)ppm.

Example 28

0.50 g (0.002 mol) of 4-octylthiobenzaldehyde and 0.28 g (0.002 mol) of 4′-fluoroacetophenone were charged in accordance with Example 21 and the reaction and purification were similarly performed in accordance with Example 21 to produce 0.46 g of a purified product of 4-octylthio-4′-fluorochalcone with a

yield rate of 64 %. The melting point of the product was 66.0° C following shows the results of mass spectrometry and $^1$H-NMR.

| Mass Spectrometry (m/Z) | | |
|---|---|---|
| $M^+$ | (370) | 100.0 |
| M + 1 | (371) | 27.1 |
| M + 2 | (372) | 8.1 |
| M + 3 | (373) | 1.7 |

$^1$H - NMR ($CDCl_3$)
δ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
7.2 - 8.0 (m, 10H, aryl, alkene)ppm.

Example 29

0.50 g (0.002 mol) of 4-octylthiobenzaldehyde and 0.49 g (0.002 mol) of 4′-iodoacetophenone were charged in accordance with Example 21 and the reaction and purification were similarly performed in accordance with Example 21 to produce 0.46 g of a purified product of 4-octylthio-4′-iodochalcone with a yield rate of 51 %. The melting point of the product was 102.5° C. The following shows the results of mass spectrometry and $^1$H-NMR.

| Mass Spectrometry (m/Z) | | |
|---|---|---|
| $M^+$ | (478) | 100.0 |
| M + 1 | (479) | 27.1 |
| M + 2 | (480) | 8.3 |
| M + 3 | (481) | 1.5 |

$^1$H - NMR ($CDCl_3$)
δ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
7.2 - 8.0 (m, 10H, aryl, alkene)ppm.

Example 30

0.50 g (0.002 mol) of 4-octylthiobenzaldehyde and 0.31 g (0.002 mol) of 3′-chloroacetophenone were charged in accordance with Example 21 and the reaction and purification were similarly performed in accordance with Example 21 to produce 0.42 g of a purified product of 4-octylthio-3′-chlorochalcone with a yield rate of 54 %. The melting point of the product was 69.5° C. The following shows the results of mass spectrometry and $^1$H-NMR.

| Mass Spectrometry (m/Z) | | |
|---|---|---|
| $M^+$ | (386) | 100.0 |
| M + 1 | (387) | 27.1 |
| M + 2 | (388) | 41.0 |
| M + 3 | (389) | 10.3 |
| M + 4 | (390) | 3.0 |

$^1H$ - NMR ($CDCl_3$)
$\delta$ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
7.2 - 8.0 (m, 10H, aryl, alkene)ppm.

Example 31

0.50 g (0.002 mol) of 3-octylthiobenzaldehyde and 0.28 g (0.002 mol) of 4′-fluoroacetophenone were charged in accordance with Example 21 and the reaction and purification were similarly performed in accordance with Example 21 to produce 0.46 g of a purified product of 3-octylthio-4′-fluorochalcone with a yield rate of 64 %. The melting point of the product was 65.8° C. The following shows the results of mass spectrometry and $^1H$-NMR.

| Mass Spectrometry (m/Z) | | |
|---|---|---|
| $M^+$ | (370) | 100.0 |
| M + 1 | (371) | 26.9 |
| M + 2 | (372) | 8.1 |
| M + 3 | (373) | 1.3 |

$^1H$ - NMR ($CDCl_3$)
$\delta$ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
7.2 - 8.0 (m, 10H, aryl, alkene)ppm.

Example 32

0.50 g (0.002 mol) of 3-octylthiobenzaldehyde and 0.49 g (0.002 mol) of 4′-iodoacetophenone were charged in accordance with Example 21 and the reaction and purification were similarly performed in accordance with Example 21 to produce 0.49 g of a purified product of 3-octylthio-4′-iodochalcone with a yield rate of 51 %. The melting point of the product was 78.8° C. The following shows the results of mass spectrometry and $^1H$-NMR.

| Mass Spectrometry (m/Z) | | |
|---|---|---|
| $M^+$ | (478) | 100.0 |
| M + 1 | (479) | 27.1 |
| M + 2 | (480) | 8.3 |
| M + 3 | (481) | 1.5 |

$^1H$ - NMR ($CDCl_3$)
$\delta$ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
7.2 - 8.0 (m, 10H, aryl, alkene)ppm.

Example 33

0.50 g (0.002 mol) of 3-octylthiobenzaldehyde and 0.32 g (0.002 mol) of 4′-dimethylacetophenone were charged in accordance with Example 21 and the reaction and purification were similarly performed in

accordance with Example 21 to produce 0.40 g of a purified product of 3-octylthio-4′-dimethylaminochalcone with a yield rate of 51 %. The melting point of the product was 87.9° C. The following shows the results of mass spectrometry and $^1H$-NMR.

| Mass Spectrometry (m/Z) | | |
|---|---|---|
| $M^+$ | (395) | 100.0 |
| M + 1 | (396) | 29.7 |
| M + 2 | (397) | 8.9 |
| M + 3 | (398) | 1.7 |

$^1H$ - NMR ($CDCl_3$)
δ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
3.0 (S, 6H, ArN - $(CH_3)2$), 7.2 - 8.0 (m, 10H, aryl, alkene)ppm.

Example 34

0.50 g (0.002 mol) of 3-octylthiobenzaldehyde and 0.27 g (0.002 mol) of 4′-aminoacetophenone were charged into a reaction vessel with 20 ml of ethanol and stirred at 20° C. A mixed solution of 1 g of a 40%-aqueous solution of sodium hydroxide and 10 ml of ethanol was added dropwise into the reaction vessel. After termination of the dropwise addition, the reaction was carried out for 30 minutes while the reaction temperature was maintained at 20° C. To the reaction product was then added 30 ml of distilled water and stirred to terminate the reaction. The resulting product was extracted with benzene, washed several times with water, dried over anhydrous magnesium sulfate and condensed to a crude product.

The crude product was fractionated by an ODS column/methanol-water solvent system liquid chromatography to produce 0.37 g of a purified product of 3-octylthio-4′-aminochalcone with a yield rate of 50 %. The following shows the results of mass spectrometry and $^1H$-NMR.

| Mass Spectrometry (m/Z) | | |
|---|---|---|
| $M^+$ | (367) | 100.0 |
| M + 1 | (368) | 27.3 |
| M + 2 | (369) | 8.3 |
| M + 3 | (370) | 1.5 |

$^1H$ - NMR ($CDCl_3$)
δ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
3.0 (s, 6H, ArN - $(CH_3)2$),
7.2 - 8.0 (m, 10H, aryl, alkene)ppm.

Example 35

0.50 g (0.002 mol) of 3-octylthiobenzaldehyde and 0.33 g (0.002 mol) of 4′-nitroacetophenone were charged into a reaction vessel with 20 ml of ethanol and stirred at 20° C. A mixed solution of 1 g of a 40%-aqueous solution of sodium hydroxide and 10 ml of ethanol was added dropwise into the reaction vessel. After termination of the dropwise addition, the reaction was carried out for 30 minutes while the reaction temperature was maintained at 20° C. To the reaction product was then added 20 ml of a 0.5 N aqueous solution of hydrochloric acid and stirred to terminate the reaction. The resulting product was extracted with benzene, washed several times with water, dried over anhydrous magnesium sulfate and condensed to a crude product.

The crude product was fractionated by an ODS column/methanol-water solvent system liquid chromatography to produce 0.32 g of a purified product of 3-octylthio-4′-nitrochalcone with a yield rate of 40 %. The following shows the results of mass spectrometry and $^1H$-NMR.

| Mass Spectrometry (m/Z) | | |
|---|---|---|
| $M^+$ | (397) | 100.0 |
| M + 1 | (398) | 27.5 |
| M + 2 | (399) | 8.7 |
| M + 3 | (400) | 1.7 |

$^1H$ - NMR ($CDCl_3$)
$\delta$ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
3.0 (s, 6H, ArN - $(CH_3)_2$),
7.2 - 8.0 (m, 10H, aryl, alkene)ppm.

Example 36

0.50 g (0.002 mol) of 3-octylthiobenzaldehyde and 0.30 g (0.002 mol) of 3′-chloroacetophenone were charged in accordance with Example 35 and the reaction and purification were similarly performed in accordance with Example 35 to produce 0.38 g of a purified product of 3-octylthio-3′-chlorochalcone with a yield rate of 50 %. The following shows the results of mass spectrometry and $^1H$-NMR.

Mass Spectrometry (m/Z)

| | | |
|---|---|---|
| $M^+$ | (386) | 100.0 |
| M + 1 | (387) | 27.1 |
| M + 2 | (388) | 40.7 |
| M + 3 | (389) | 10.3 |
| M + 4 | (390) | 3.0 |

$^1H$ - NMR ($CDCl_3$)
$\delta$ 0.8 - 1.1 (tri, 3H, $ArSCH_2(CH_2)_6$ - $CH_3$),
1.3 - 1.8 (b, 12H, $ArSCH_2$ - $(CH_2)_6$ - $CH_3$),
2.9 - 3.2 (tri, 3H, ArS - $CH_2$ - $(CH_2)_6CH_3$),
3.0 (s, 6H, ArN - $(CH_3)_2$),
7.2 - 8.0 (m, 10H, aryl, alkene)ppm.

Example 37

1.66 g (0.01 mol) of 4′-methylthioacetophenone and 1.85 g (0.01 mol) of 3-bromobenzaldehyde were charged into a reaction vessel with 20 ml of ethanol and stirred at 20° C. A mixed solution of 1 g of a 40%-aqueous solution of sodium hydroxide and 10 ml of ethanol was added dropwise into the reaction vessel. After termination of the dropwise addition, the reaction was carried out for 30 minutes while the reaction

temperature was maintained at 20 °C. To the reaction product was then added 20 ml of a 0.5 N aqueous solution of hydrochloric acid and stirred to terminate the reaction. A separated solid was filtered, washed several times with water and dried.

The resulting crude product was recrystallized with an ethanol solvent to produce 2.36 g of a purified product of 3-bromo-4′-methylthiochalcone with a yield rate of 71 %. The melting point of the product was 180 °C. Fig.4 shows the $^1$H-NMR chart. The following shows the results of mass spectrometry and $^1$H-NMR.
$^1$H - NMR ($CDCl_3$)
δ 2.5 (S, 3H, ArS - $CH_3$),
7.2 - 8.0 (m, 10H, aryl, alkene)ppm.

| Mass Spectrometry (m/Z) | | |
|---|---|---|
| $M^+$ | (332) | 100.0 |
| M + 1 | (333) | 18.9 |
| M + 2 | (334) | 104.1 67 |
| M + 3 | (335) | 19.5 |
| M + 4 | (336) | 6.3 |

Example 38

1.66 g (0.01 mol) of 4′-methylthioacetophenone and 1.85 g (0.01 mol) of 4-bromobenzaldehyde were charged in accordance with Example 37 and the reaction and purification were similarly performed in accordance with Example 37 to produce 2.60 g of a purified product of 4-bromo-4′-methylthiochalcone with a yield rate of 78 %. The melting point of the product was 180 °C. The following shows the results of mass spectrometry and $^1$H-NMR.
$^1$H - NMR ($CDCl_3$)
δ 2.5 (S, 3H, ArS - $CH_3$),
7.2 - 8.1 (m, 10H, aryl, alkene)ppm.

| Mass Spectrometry (m/Z) | | |
|---|---|---|
| $M^+$ | (332) | 100.0 |
| M + 1 | (333) | 18.9 |
| M + 2 | (334) | 104.0 |
| M + 3 | (335) | 18.7 |
| M + 4 | (336) | 6.0 |

Example 39

1.66 g (0.01 mol) of 4′-methylthioacetophenone and 1.24 g (0.01 mol) of 4-fluorobenzaldehyde were charged in accordance with Example 37 and the reaction and purification were similarly performed in accordance with Example 37 to produce 1.90 g of a purified product of 4-fluoro-4′-methylthiochalcone with a yield rate of 70 %. The melting point of the product was 126 °C. The following shows the results of mass spectrometry and $^1$H-NMR.
$^1$H - NMR ($CDCl_3$)
δ 2.5 (S, 3H, ArS - $CH_3$),
7.0 - 8.0 (m, 10H, aryl, alkene)ppm.

| Mass Spectrometry (m/Z) | | |
|---|---|---|
| $M^+$ | (272) | 100.0 |
| M + 1 | (273) | 19.0 |
| M + 2 | (274) | 6.3 |

Example 40

1.66 g (0.01 mol) of 4′-methylthioacetophenone and 3.74 g (0.01 mol) of 4-octadecyloxybenzaldehyde were charged in accordance with Example 37 and the reaction and purification were similarly performed in accordance with Example 37 to produce 3.40 g of a purified product of 4-octadecyloxy-4′-methylthiochalcone with a yield rate of 65 %. The melting point of the product was 111 °C. The following shows the results of mass spectrometry and $^1H$-NMR.

$^1H$ - NMR ($CDCl_3$)
δ 0.7 - 1.9 (m, 35H, alkane),
2.5 (S, 3H, ArS - $CH_3$),
3.9 - 4.1 (t, ArO - $\underline{CH_2}$ - $C_{17}H_{35}$),
6.8 - 8.1 (m, 10H, aryl, alkene)ppm.

| Mass Spectrometry (m/Z) | | |
|---|---|---|
| $M^+$ | (522) | 100.0 |
| M + 1 | (523) | 40.0 |
| M + 2 | (524) | 12.4 |
| M + 3 | (525) | 2.9 |

Example 41

0.27 g (0.0025 mol) of benzaldehyde and 0.52 g (0.0025 mol) of 4′-butylthioacetophenone were charged into a reaction vessel with 20 ml of ethanol and stirred at 20 °C. A mixed solution of 1 g of a 40%-aqueous solution of sodium hydroxide and 10 ml of ethanol was added dropwise into the reaction vessel. After termination of the dropwise addition, the reaction was carried out for 30 minutes while the reaction temperature was maintained at 20 °C. To the reaction product was then added 20 ml of a 0.5 N aqueous solution of hydrochloric acid and stirred to terminate the reaction. A separated solid product was filtered, washed several times with water and dried.

The resulting crude product was recrystallized with an ethanol solvent to produce 0.44 g of a purified product of 4′-butylthiochalcone with a yield rate of 60 %. The melting point of the product was 77.0 °C. The following shows the results of mass spectrometry and $^1H$-NMR.

| Mass Spectrometry (m/Z) | | |
|---|---|---|
| $M^+$ | (296) | 100.0 |
| M + 1 | (297) | 22.1 |
| M + 2 | (298) | 6.9 |
| M + 3 | (299) | 1.0 |

$^1H$ - NMR ($CDCl_3$)
δ 0.8 - 1.1 (tri, 3H, $ArSCH_2CH_2CH_2$ - $CH_3$),
1.3 - 1.8 (m, 4H, $ArSCH_2$ - $CH_2CH_2$- $CH_3$),
2.9 - 3.2 (tri, 3H, ArS - $CH_2$ - $CH_2CH_2CH_3$),

7.2 - 8.0 (m, 11H, aryl, alkene)ppm.

## Claims

1. A chalcone derivative compound represented by the following general formula (I)

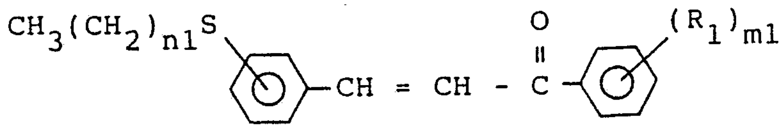

... (I)

wherein $R_1$ represents a halogen atom, a hydroxyl group, an amino group, a dimethylamino group, a nitro group, a cyano group, a phenyl group, an acetyl group, an alkyl group having 1 to 18 carbon atoms or an alkyloxy group having 1 to 22 carbon atoms, $n_1$ represents an integer of 0 to 21 and $m_1$ represents an integer of 0 to 5.

2. The chalcone derivative compound according to claim 1 wherein the chalcone derivative compound represented by the formula (I) is selected from the group consisting of 4-methylthiochalcone, 4-methylthio-4′-chlorochalcone, 4-methyltio-4′-bromochalcone, 4-methylthio-4′-hydroxychalcone, 4-methylthio-4′-aminochalcone, 4-methylthio-4′-nitrochalcone, 4-methylthio-4′-dimethylaminochalcone, 4-methylthio-4′-methoxychalcone, 4-methylthio-4′-ethoxychalcone, 4-methylthio-4′-octadecyloxychalcone, 4-methylthio-3′-bromochalcone, 4-methylthio-3′-methylchalcone, 4-methylthio-3′-methoxychalcone, 4-methylthio-3′-aminochalcone, 4-methylthio-3′-dimethylaminochalcone, 4-methylthio-3′-nitrochalcone, 4-methyl thio-2′-nitrochalcone, 4-methylthio-3′,4′-dimethoxychalcone, 4-methylthio-3′,4′,5′-trimethoxychalcone, 4-methylthio-4′-phenylchalcone, 4 methylthio-4′-acetylchalcone, 4-methylthio-3′-chlorochalcone, 4-ethylthio-3′-chlorochalcone, 4-ethylthio-4′-bromochalcone, 4-ethylthio-4′-iodochalcone, 4-butylthiochalcone, 4-butylthio-3′-chlorochalcone, 4-butylthio-4′-bromochalcone, 4-butylthio-4′-iodochalcone, 4-octylthio-4′-fluorochalcone, 4-octylthio-4′-iodochalcone, 4-octylthio-3′-chlorochalcone, 3-octylthio-4′-fluorochalcone, 3-octylthio-4′-iodochalcone, 3-octylthio-4′-dimethylaminochalcone, 3-octylthio-4′-aminochalcone, 3-octylthio-4′-nitrochalcone and 3-octylthio-3′-chlorochalcone.

3. The chalcone derivative compound according to claim 1 wherein the chalcone derivative compound represented by the general formula (I) is obtained by a condensation reaction between alkylthiobenzaldehyde and acetophenone or a derivative thereof.

4. The chalcone derivative compound according to claim 3 wherein said condensation reaction is carried out in the temperature range of between 0° and 50° C.

5. A chalcone derivative compound represented by the following general formula (II)

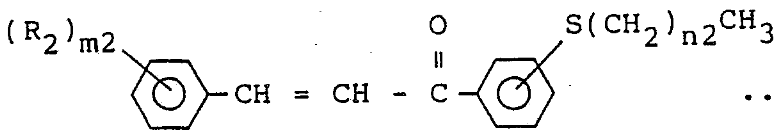

... (II)

wherein R2 represents a halogen atom, a hydroxyl group, an amino group, a dimethylamino group, a nitro group, a cyano group, a phenyl group, an acetyl group, an alkyl group having 1 to 18 carbon atoms or an alkyloxy group having 1 to 22 carbon atoms, $n_2$ represents an integer of 0 to 21 and $m_2$ represents an integer of 0 to 5.

6. The chalcone derivative compound according to claim 5 wherein the chalcone derivative compound represented by the general formula (II) is selected from the group consisting of 3-bromo-4′-methylthiochalcone, 4-bromo-4′-methylthiochalcone, 4-fluoro-4′-methylthiochalcone, 4-octadecyloxy-4′-methylthiochalcone and 4′-butylthiochalcone.

7. The chalcone derivative compound according to claim 5 wherein the chalcone derivative compound represented by the general formula (II) is obtained by a condensation reaction between alkylthioacetophenone and benzaldehyde or a derivative thereof.

8. The chalcone derivative compound according to claim 7 wherein said condensation reaction is carried out in the temperaturte range of from 0° to 50° C.

FIG.1

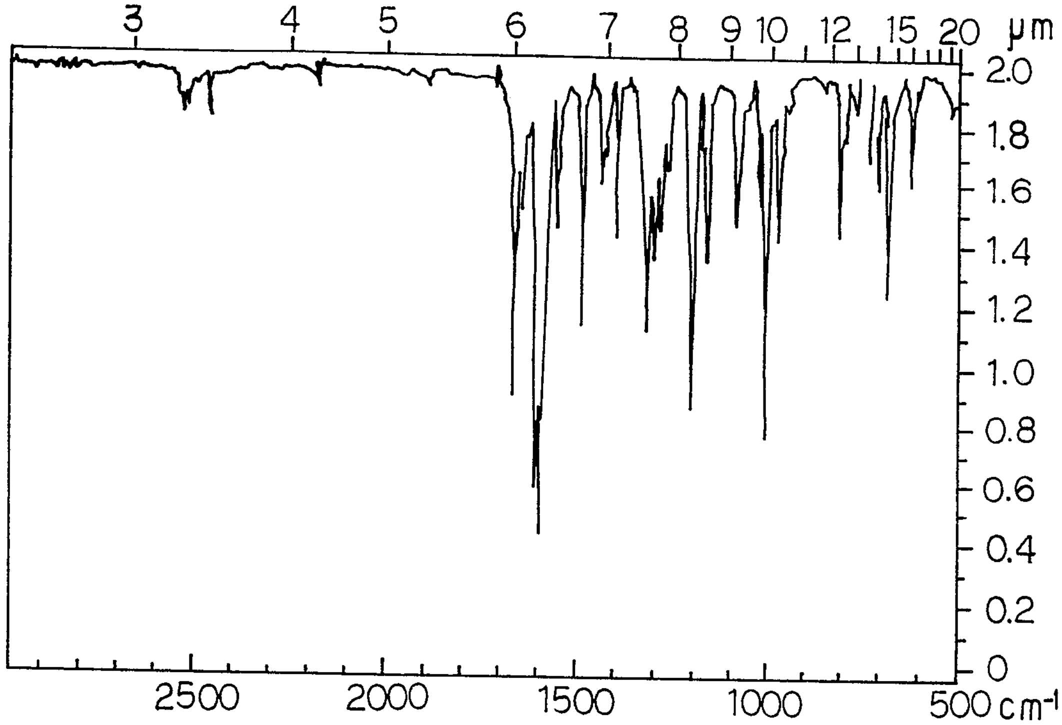
3
4
5
6
7
8
9
10
12
15
20
μm
2.0
1.8
1.6
1.4
1.2
1.0
0.8
0.6
0.4
0.2
0
2500
2000
1500
1000
500 cm-1

FIG.2

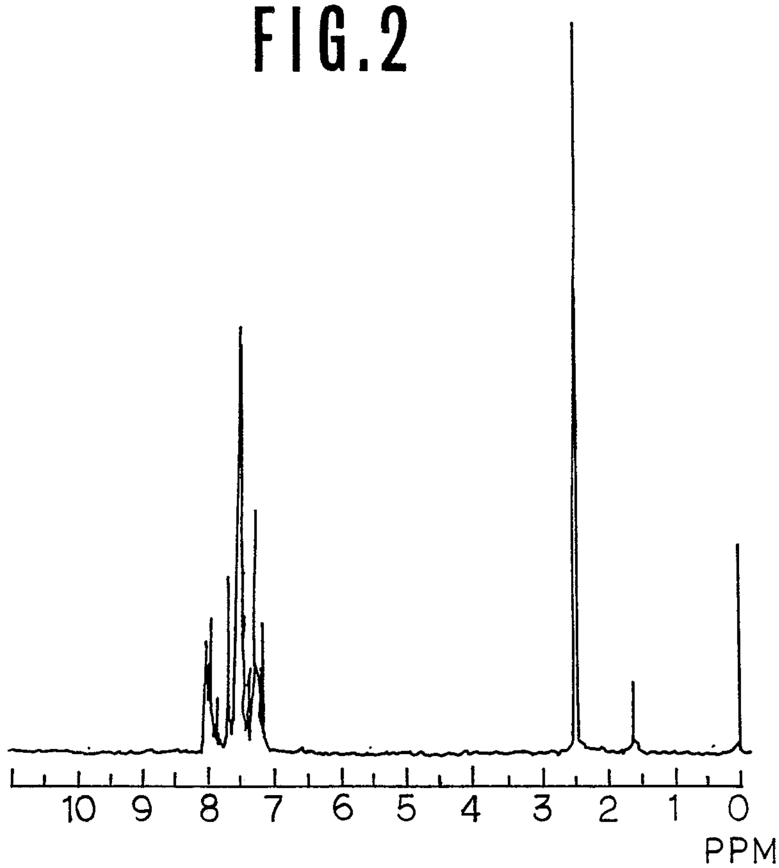
10
9
8
7
6
5
4
3
2
1
0
PPM

FIG.3

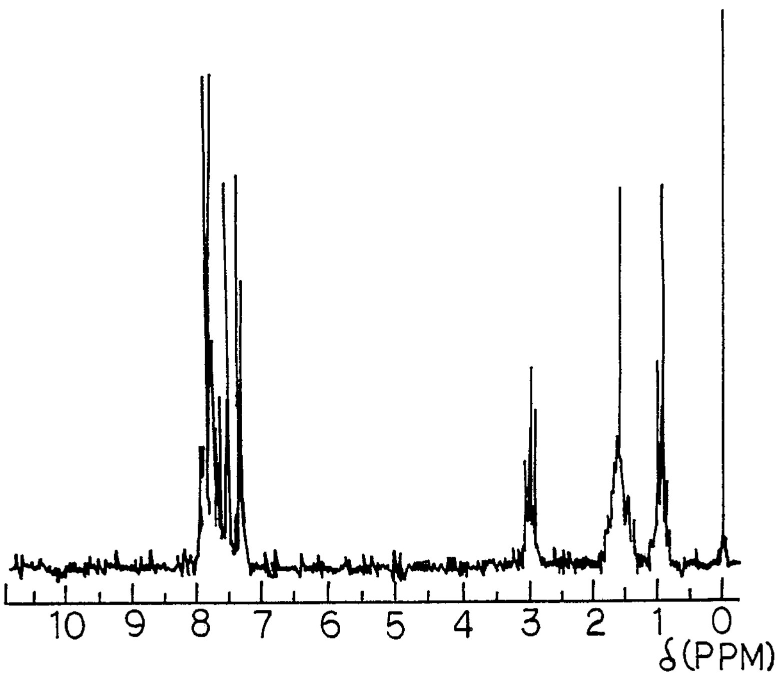


FIG.4

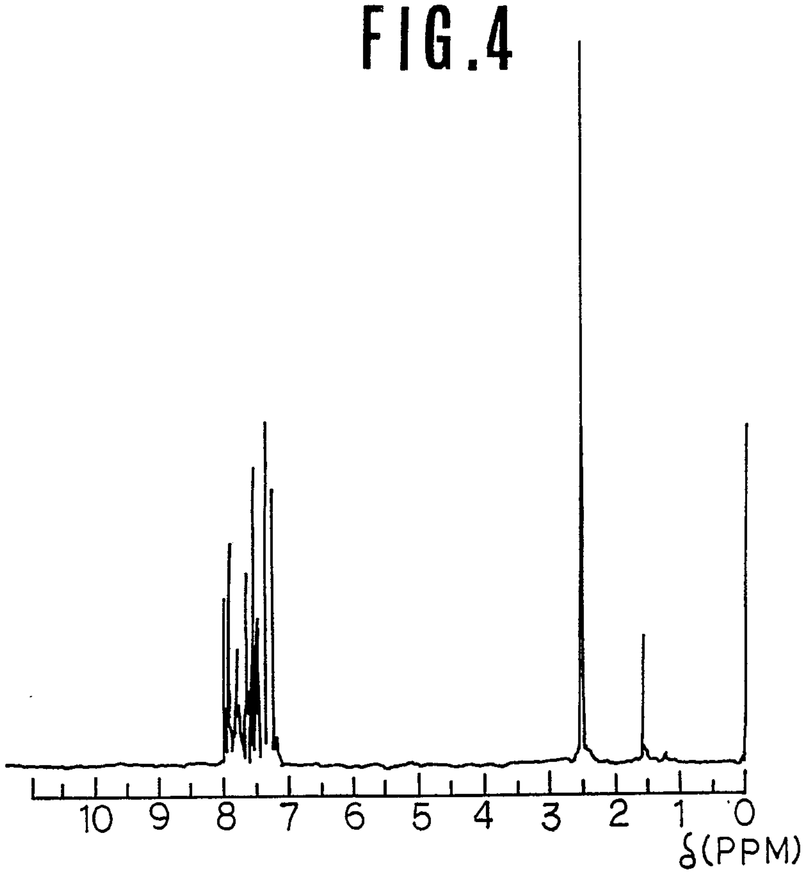

# INTERNATIONAL SEARCH REPORT

International Application No PCT/JP88/00744

**I. CLASSIFICATION OF SUBJECT MATTER** (if several classification symbols apply, indicate all) [6]

According to International Patent Classification (IPC) or to both National Classification and IPC

Int.Cl$^4$ C07C149/32

**II. FIELDS SEARCHED**

Minimum Documentation Searched [7]

| Classification System | Classification Symbols |
|---|---|
| IPC | C07C149/32, A61K31/12 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 51-125736 (Rikagaku Kenkyusho) 2 November 1976 (02. 11. 76) Columns 1 to 4 (Family: none) | 1-8 |
| X | Yakugaku Zasshi, Vol. 91, No.8 August 1971 (Tokyo), Hirose Kazuo and two others [Ganryu Chalcone Yudotai no Gosei narabini sono Seibutsu Kassei ni tsuite (Report No.1) Alkylthiochalcone rui no Gosei narabini Kokin Sayo] P804-810 | 1-8 |
| X | JP, A, 50-106960 (American Cyanamid Co.) 22 August 1975 (22. 08. 75) Columns 1 to 4 & DE, A1, 2502490 | 1-6 |
| X | JP, A, 56-7736 (F. Hoffmann - La Roche & Co., A.G.) 27 January 1981 (27. 01. 81) Columns 1 to 9 & EP, A1, 13960 | 1-8 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 29, 1988 (29. 09. 88) | October 11, 1988 (11. 10. 88) |
| **International Searching Authority** | **Signature of Authorized Officer** |
| Japanese Patent Office | |

**FURTHER INFORMATION CONTINUED FROM THE SECOND SHEET**

| | | |
|---|---|---|
| X | JP, A, 57-122039 (F. Hoffmann - La Roche & Co., A.G.)<br>29 July 1982 (29. 07. 82)<br>Columns 1 to 10 & EP, A1, 51819 | 1-8 |

**V. ☐ OBSERVATIONS WHERE CERTAIN CLAIMS WERE FOUND UNSEARCHABLE [10]**

This international search report has not been established in respect of certain claims under Article 17(2) (a) for the following reasons:

1. ☐ Claim numbers............ because they relate to subject matter [12] not required to be searched by this Authority, namely:

2. ☐ Claim numbers............ because they relate to parts of the international application that do not comply with the prescribed requirements to such an extent that no meaningful international search can be carried out [13], specifically:

**VI. ☐ OBSERVATIONS WHERE UNITY OF INVENTION IS LACKING [11]**

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.